# FASCICULE DE BREVET EUROPEEN

(11) **EP 0 785 789 B1**
(45) Date de publication et mention de la délivrance du brevet: **11.09.2002**
(21) Numéro de dépôt: 95935495.2
(22) Date de dépôt: 13.10.1995
(51) Int. Cl.: A61K 31/55, C07D 487/06

(54) **DIAZEPINO-INDOLES INHIBITEURS DE PHOSPHODIESTERASES IV**
DIAZEPINOINDOLE INHIBITOREN VON PHOSPHODIESTERASE IV
DIAZEPINO-INDOLES AS PHOSPHODIESTERASE IV INHIBITORS

(30) Priorité: 14.10.1994 FR 9412282
(43) Date de publication de la demande: 30.07.1997
(73) Titulaire: Pfizer, 75014 Paris (FR)
(72) Inventeur: PASCAL, Yves, F-92500 Rueil-Malmaison (FR); MOODLEY, Indres, Craighall 2024 (ZA); CALVET, Alain, F-94240 L'Hay-les-Roses (FR); JUNIEN, Jean-Louis, F-92310 Sèvres (FR); DAHL, Svein, G., F-91190 Gif-sur-Yvette (FR)
(74) Mandataire: Dufresne, Guillaume Alain François
(86) Numéro de dépôt international: FR9501354
(87) Numéro de publication internationale: WO96011690

(56) Documents cités:
- EP-A- 0 265 734

## Description

### Domaine de l'invention

La présente invention est relative à l'utilisation de [1,4]diazépino[6,7,1-*hi*]indoles dont certains sont nouveaux, pour la préparation de médicaments permettant de traiter des affections relevant d'une thérapie par un inhibiteur de phosphodiestérases IV. Ces médicaments sont utiles notamment comme anti-inflammatoires, anti-allergiques, bronchodilatateurs, ou anti-asthmatiques, et sont dénués d'effets secondaires digestifs ou cardiaques.

### Arrière-plan technologique de l'invention

Différemment des propriétés révélées par la présente invention, l'art antérieur fait état de [1,4]diazépino[6,7,1-*hi*]indoles pour lesquels il est décrit des propriétés antagonistes de la cholécystokinine (CCK) et/ou de la gastrine, et qui sont proposés pour des affections du tube digestif : estomac, intestin, pancréas et vésicule biliaire, et notamment les troubles de la satiété.

Ainsi, la demande de brevet européen n° 340 064 décrit des composés de formule : 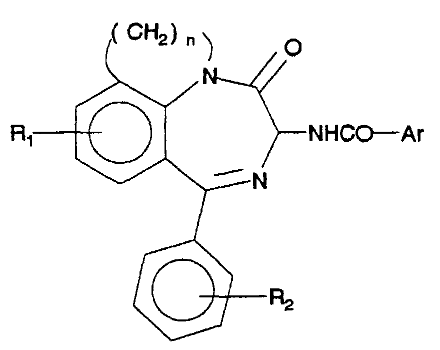 dans laquelle R₁ et R₂ sont hydrogène ou halogène, Ar est indolyle ou phényle et n est 2 ou 3. Ces composés sont des antagonistes périphériques de la cholécystokinine (CCK_{A}).

La demande de brevet européen n° 360 079 décrit des composés antagonistes périphériques et/ou centraux de la CCK de formule : 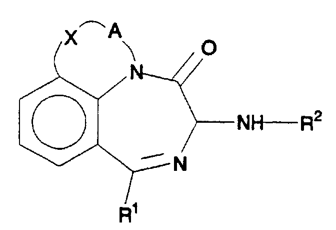 dans laquelle R¹ est aryle éventuellement substitué, X est oxygène ou méthylène éventuellement substitué par un radical alkyle inférieur, A est une liaison ou alkylène inférieur qui peut avoir un ou des groupes alkyles inférieurs, R² est hydrogène, ou acyle dans un sens excessivement large. En fait il apparaît que les produits préférés de cette demande EP 360 079 sont ceux dans lesquels :
- R¹ est égal à 2-fluorophényle, et/ou
- R² est un groupe aryl-propenoyl ou hétéroaryl-propenoyl, et/ou
- la configuration du carbone de la diazépine en α de son carbonyle est (S), ou (R, S).

En ce qui concerne l'inhibition des phosphodiestérases, il est rappelé que l'adénosine 3', 5'-monophosphate cyclique (AMPc) est un second messager intracellulaire ubiquitaire, intermédiaire entre un premier messager (hormone, neurotransmetteur, ou autacoïde) et les réponses fonctionnelles cellulaires : le premier messager stimule l'enzyme responsable de la synthèse de l'AMPc ; l'AMPc intervient alors, selon les cellules en cause, dans de très nombreuses fonctions : métaboliques, contractiles, ou sécrétoires.

Les effets de l'AMPc prennent fin lorsqu'il est dégradé par les phosphodiestérases des nucléotides cycliques, enzymes intracellulaires qui catalysent son hydrolyse en adénosine 5'-monophosphate inactive.

On distingue chez les mammifères au moins cinq grandes familles de phosphodiestérases des nucléotides cycliques (PDE) numérotées de _{I} à _{V}, selon leur structure, leur comportement cinétique, leur spécificité de substrat, ou leur sensibilité à des effecteurs (Beavo J.A.(1990) Trends Pharmacol. Sci. 11, 150-155). Les PDE _{IV} sont spécifiques de l'AMPc.

Des composés inhibiteurs non spécifiques de phosphodiestérases sont connus, qui inhibent plusieurs familles d'enzymes. C'est le cas de certaines méthylxanthines comme la théophylline. Ces composés ont un index thérapeutique faible, notamment en raison de leur action sur des types de PDE présents dans des cellules autres que les cellules cibles. A l'inverse, certaines familles de PDE peuvent être inhibées sélectivement par divers agents pharmacologiques : l'hydrolyse des nucléotides cycliques est ralentie et donc leur concentration augmente dans les seules cellules où se trouve le type de PDE sensible à l'inhibiteur.

Un intérêt particulier se manifeste pour les phosphodiestérases _{IV} (PDE _{IV}), qui ont été identifiées dans de nombreux tissus dont le système nerveux central, le coeur, l'endothélium vasculaire, le muscle lisse vasculaire et celui des voies aériennes, les lignées myéloïdes et lymphoïdes.

Une augmentation de l'AMPc dans les cellules impliquées dans l'inflammation inhibe leur activation : inhibition de la synthèse et de la libération de médiateurs au niveau des mastocytes, des monocytes, des polynucléaires éosinophiles et basophiles, inhibition du chimiotactisme et de la dégranulation des polynucléaires neutrophiles et éosinophiles, inhibition des divisions et de la différenciation des lymphocytes.

Les cytokines, notamment TNF et interleukines, produits par différents types de leukocytes comme les lymphocytes T et les polynucléaires éosinophiles, jouent un rôle important dans le déclenchement des manifestations inflammatoires en particulier en réponse à une stimulation par un allergène au niveau des voies respiratoires.

D'autre part, l'AMPc diminue le tonus des fibres musculaires lisses des voies aériennes ; les inhibiteurs de PDE _{IV} déterminent une bronchorelaxation.

On peut donc s'attendre à ce que des inhibiteurs sélectifs de PDE _{IV} possèdent une activité thérapeutique comme médicaments anti-inflammatoires, anti-allergiques, bronchodilatateurs, et dans le traitement de l'asthme, où l'on observe une infiltration des voies aériennes par des cellules inflammatoires et une bronchoconstriction.

La théophylline est très largement utilisée depuis longtemps dans le traitement de l'asthme, et bien que son mécanisme d'action soit complexe, l'inhibition de PDE contribue à son action, mais aussi à certains effets indésirables comme les nausées et les céphalées.

Cependant jusqu'ici la mise au point d'inhibiteurs puissants de PDE _{IV} s'est avérée difficile du fait que beaucoup des inhibiteurs potentiels de PDE _{IV} ne sont pas dénués d'activité sur les phosphodiestérases des autres familles.

Le manque de sélectivité des inhibiteurs de PDE _{IV} représente donc un problème, étant donnée l'étendue des fonctions régulées par l'AMPc. Il existe donc un besoin pour des inhibiteurs puissants et sélectifs de PDE _{IV}, c'est-à-dire n'ayant pas d'action vis-à-vis des PDE appartenant à d'autres familles.

Le rolipram (DCI), dérivé de pyrrolidone synthétisé dès 1975, est considéré comme représentatif des inhibiteurs spécifiques de PDE _{IV}. De nombreux composés apparentés au rolipram ont été synthétisés en vue de leur utilisation comme inhibiteurs de PDE _{IV}. In vitro, le rolipram inhibe l'activité des cellules inflammatoires chez les rongeurs : inhibition de la synthèse des médiateurs par les mastocytes, les polynucléaires éosinophiles et basophiles, et les monocytes ; inhibition du chimiotactisme et de la dégranulation des polynucléaires. Le rolipram a été proposé comme antidépresseur ; cependant son utilisation s'accompagne d'effets indésirables à type de nausées et de vomissements.

### Sommaire de l'invention

Or, rompant avec l'état de la technique, on a maintenant trouvé que des dérivés de [1,4]diazépino[6,7,1-*hi*]indoles, dont certains sont nouveaux, sont de façon surprenante de puissants inhibiteurs de PDE _{IV} à des concentrations auxquelles ils ont peu ou pas d'action sur les autres familles de PDE.

L'invention concerne essentiellement l'utilisation de diazépino-indoles, dont certains sont nouveaux, de formule (I) 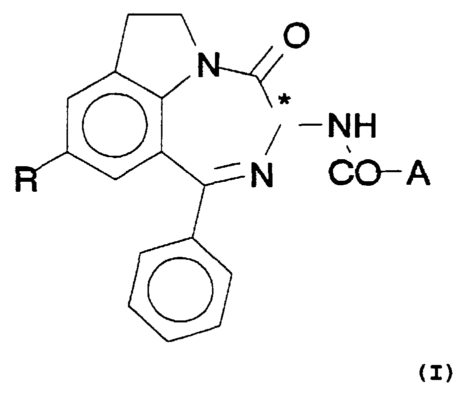 dans laquelle :
- R est hydrogène, alkyle inférieur ou alkoxy inférieur,
- A est aryle, hétéroaryle azoté, ou hétéroaryle soufré, chacun éventuellement substitué par un à trois groupes indépendamment choisis parmi halogène, alkyle inférieur, haloalkyle, alkoxy inférieur, hydroxy, acétoxy, amino, *t*-butoxycarbonylamino, cycloalkylcarbonylamino, ou acétamido.

### Description détaillée de l'invention

Dans un premier aspect, l'invention vise l'utilisation, pour la préparation de médicaments permettant de traiter des affections relevant d'une thérapie par un inhibiteur de phosphodiestérases _{IV}, des diazépino-indoles de formule (I) 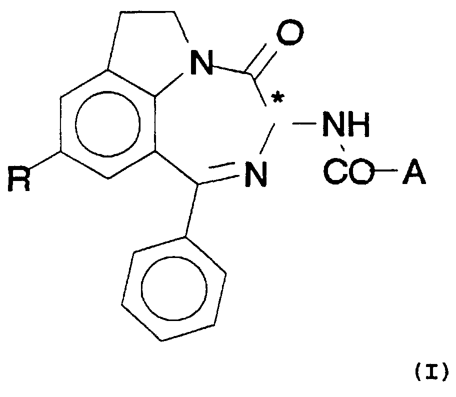 dans laquelle :
- R est hydrogène, alkyle inférieur ou alkoxy inférieur ;
- A est aryle, hétéroaryle azoté, ou hétéroaryle soufré, chacun éventuellement substitué par un à trois groupes indépendamment choisis parmi halogène, alkyle inférieur, haloalkyle, alkoxy inférieur, hydroxy, acétoxy, amino, *t*-butoxycarbonylamino, cycloalkylcarbonylamino, ou acétamido ; de leurs formes racémiques, de leurs isomères de configuration déterminée par le carbone en position 3 du noyau diazépino-indol-4-one, et de leurs sels pharmacologiquement acceptables.

Dans un second aspect, l'invention vise les diazépino-indoles de formule (I') 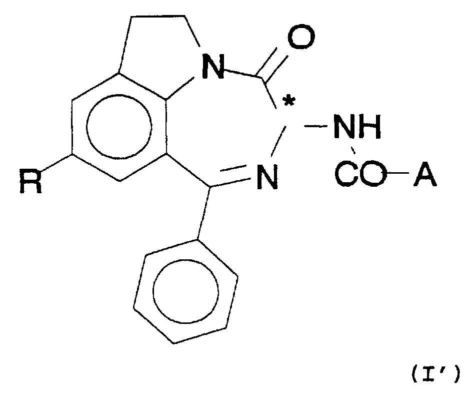 dans laquelle :
- R est hydrogène, alkyle inférieur ou alkoxy inférieur ;
- A est aryle, hétéroaryle azoté, ou hétéroaryle soufré, chacun éventuellement substitué par un à trois groupes indépendamment choisis parmi halogène, alkyle inférieur, haloalkyle, alkoxy inférieur, hydroxy, acétoxy, amino, *t*-butoxycarbonylamino, cycloalkylcarbonylamino, ou acétamido ; leurs formes racémiques et leurs isomères de configuration déterminée par le carbone en position 3 du noyau diazépino-indol-4-one ainsi que leurs sels pharmacologiquement acceptables, sous réserve que, lorsque R est hydrogène :
   *i)* A ne représente pas le radical 2-indolyle, ni un radical phényle substitué par de un à trois groupes alkoxy ;
   *ii)* pour les formes racémiques ou de configuration (S), A ne représente pas un radical phényle substitué par un halogène, un halogène et un groupe amino, ou un groupe haloalkyle.

Dans ce qui précède ainsi que dans ce qui suit :
- par aryle on entend un groupement phényle ou naphtyle ;
- par hétéroaryle azoté on entend un groupement monocyclique ou polycyclique non saturé contenant au moins un atome d'azote et de préférence ces hétérocycles azotés peuvent être des groupements hétéromonocycliques ayant de quatre à sept sommets et contenant de 1 à 4 atomes d'azote, ou des groupements hétérocycliques condensés non saturés contenant de 1 à 4 atomes d'azote ; le groupement hétéroaryle azoté peut être méthylé ou éthylé sur un azote chargé positivement ;
- par halogène on entend le fluor, le chlore, le brome ou l'iode ;
- par alkyle inférieur on entend les groupes alkyles linéaires ou ramifiés comportant de un à quatre atomes de carbone ;
- par cycloalkyle on entend les groupes cyclopropyles, cyclobutyles et cyclopentyles ;
- par alkoxy inférieur on entend un groupe O-alkyle dans lequel le groupe alkyle est alkyle inférieur tel que défini ci-dessus ;
- par haloalkyle on entend un mono-, di-, ou trihaloalkyle contenant de 1 à 4 atomes de carbone.

On trouvera une revue des sels acceptables en pharmacie dans J. Pharm. Sci., 1977, 66, 1-19. Toutefois, par sel pharmacologiquement acceptable d'un composé de formule (I) présentant une portion basique on entend les sels d'addition des composés de formule (I) que l'on forme à partir d'acides minéraux ou organiques non toxiques comme par exemple les sels d'acides bromhydrique, chlorhydrique, sulfurique, sulfamique, phosphorique, nitrique, acétique, propionique, succinique, glycolique, stéarique, lactique, malique, tartrique, citrique, mucique, ascorbique, pamoïque, maléique, hydroxymaléique, phénylacétique, glutamique, benzoïque, salicylique, sulfanilique, acétoxybenzoïque, fumarique, toluène-sulfonique, éthanedisulfonique, oxalique, isethionique et autres. Les divers sels d'ammonium quaternaires des dérivés (I) sont également inclus dans cette catégorie des composés de l'invention. Et par sel pharmacologiquement acceptable d'un composé de formule (I) présentant une portion acide on entend les sels usuels des composés de formule (I) que l'on forme à partir de bases minérales ou organiques non toxiques comme par exemple les hydroxydes des métaux alcalins et alcalino-terreux (lithium, sodium, potassium, magnésium et calcium), les amines (dibenzyléthylènediamine, triméthylamine, pipéridine, pyrrolidine, benzylamine et autres) ou encore les hydroxydes d'ammoniums quaternaires comme l'hydroxyde de tétraméthylammonium.

Parmi les diazépino-indoles de formule (I) et (I'), on préfère ceux dans lesquels R est alkyle inférieur ou alkoxy inférieur et de préférence méthyle ou méthoxy. On préfère de manière générale les diazépino-indoles de formule (I) et (I') où l'atome de carbone asymétrique en position alpha par rapport au carbonyle du cycle diazépine possède la configuration absolue (R) (selon la nomenclature de Cahn, Ingold et Prelog), c'est-à-dire opposée à celle annoncée favorable (S) pour l'affinité de type antagoniste aux récepteurs de la CCK.

L'invention vise également un procédé de préparation de diazépino-indoles de formule (I) qui consiste à mettre à réagir une amine racémique ou optiquement active de formule (II) : 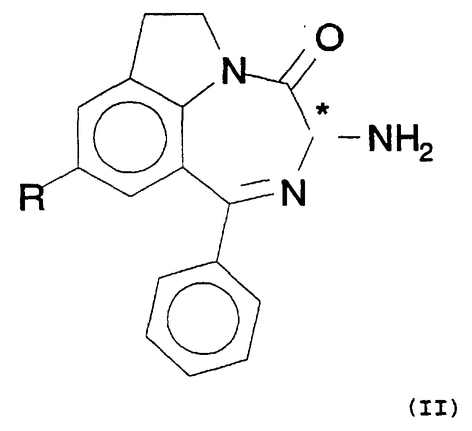 sur un dérivé d'acide carboxylique de formule (III) : 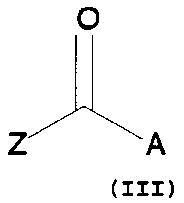 dans laquelle A a la signification indiquée ci-dessus et Z est un halogène, un groupe azido, un groupe imidazol-1-yle, un groupe -O-CO-Z₁, Z₁ pouvant être, outre A, un radical alkyle encombré comportant de 3 à 6 atomes de carbone, enfin Z₁ pouvant être un groupe O-Z₂, Z₂ étant un groupe aromatique comportant un ou deux cycles substitué par un ou plusieurs radicaux nitro ou halogènes, pour obtenir un composé de formule (I), racémique ou optiquement actif.

En particulier, on décrira la préparation de diazépino-indoles de formule (I) selon trois procédés A, B et C, dans lesquels (III) représente les produits de formule (III_{A}), (III_{B}) et (III_{C}) respectivement : 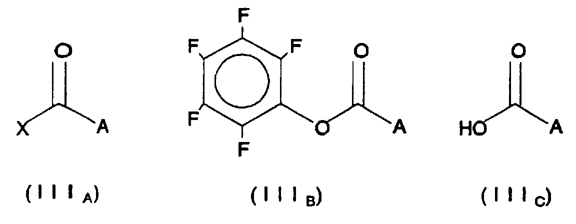 selon la réaction :

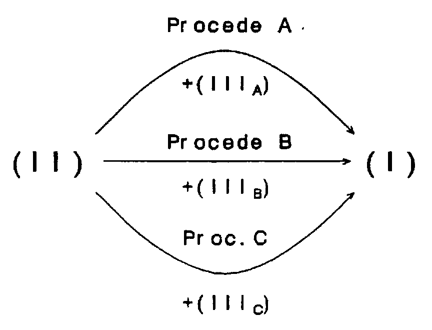

On peut notamment opérer de la manière suivante :

### Procédé A :

Un composé de formule (II) est dissous dans 5 à 50 volumes d'un solvant organique anhydre comme par exemple un hydrocarbure chloré comme du dichlorométhane ou du chloroforme, un éther-oxyde linéaire ou cyclique comme du diméthoxy-1, 2-éthane, du tétrahydrofurane ou du dioxane, un solvant polaire aprotique tel que la pyridine, du diméthylsulfoxyde ou du diméthylformamide ou tout autre solvant convenable pour y effectuer une réaction de condensation ou encore un mélange approprié de deux ou plusieurs de ces solvants, et on y ajoute un à deux équivalents d'un halogénure d'acide de formule A-CO-X, dans laquelle X est halogène, de préférence le chlore, et A a la signification définie plus haut.

Puis on ajoute une même quantité équimoléculaire d'une base minérale ou organique, de préférence la triéthylamine, et on agite à une température comprise entre - 20° C et la température d'ébullition du mélange durant une période comprise entre trente minutes et 24 heures. Le milieu réactionnel éventuellement dilué par un des solvants cités ci-dessus est alors traité successivement par une solution diluée d'un acide minéral, puis par une solution saturée d'hydrogénocarbonate de sodium, puis par de l'eau.

Après évaporation du solvant, le produit est généralement purifié par chromatographie rapide sur colonne de silice selon une méthode adaptée de Still et al. (1978) J. Org. Chem. 43 : 2923.

### Procédé B :

Stade 1 : Un composé de formule A-COOH dans lequel A a la signification définie plus haut est dissous dans 5 à 50 volumes d'un solvant organique tel que ceux décrits pour le procédé A. On y ajoute un à trois équivalents d'un composé de formule Z₂-OH dans laquelle Z₂ a la signification indiquée plus haut, les groupes Z₂ préférés étant le paranitrophényle, le dinitro-2,4-phényle, et particulièrement le pentafluorophényle, en présence d'un agent déshydratant comme une carbodiimide et éventuellement un sel de pyridinium. Les conditions de la réaction sont similaires à celles du procédé A.

Après évaporation du solvant, selon son degré de pureté déterminé par CCM, le produit est purifié par chromatographie rapide ou engagé tel que dans la réaction du stade 2.

Stade 2 : L'ester préparé au stade précédent est ajouté à un équivalent de composé (II) dissous dans l'acétate d'éthyle anhydre. Les conditions de la réaction sont similaires à celles du procédé A. Après évaporation du solvant, le produit est purifié par chromatographie rapide.

### Procédé C :

A un composé de formule (II) en solution dans 5 à 50 volumes d'un des solvants cités au procédé A on ajoute directement un léger excès de l'acide de formule A-COOH, dans laquelle A a la signification définie plus haut, en présence d'un équivalent d'un agent de condensation comme une carbodiimide N,N' disubstituée, le N,N'-carbonyldiimidazole, ou comme de préférence le tétrafluoroborate de O-[(éthoxycarbonyl)cyanométhylamino]-N,N,N',N'-tétraméthyluronium ou encore l'hexafluorophosphate de bromo-tris-pyrrolidino-phosphonium, et de deux équivalents d'une alkylamine tertiaire. Les conditions opératoires sont similaires à celles du procédé A.

Le mélange est extrait successivement par une solution diluée d'un acide minéral, une solution saturée de NaHCO₃, et de l'eau. Après évaporation du solvant, le produit est purifié par chromatographie rapide.

Le procédé général de préparation des amines intermédiaires (II) sous leurs formes racémique et/ou d'énantiomère est documenté dans l'art antérieur. Par exemple, on peut préparer une amine de formule (II) en aminant en position alpha du carbonyle un diazépino-indole de formule (V) : 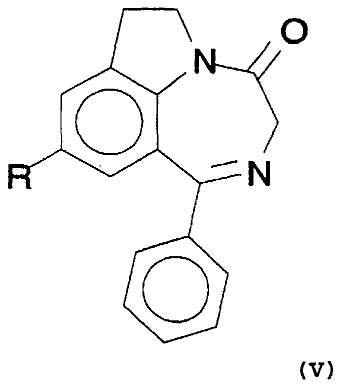 par un dérivé d'hydroxylamine ou par la chloramine ; ou encore, en deux étapes, en faisant réagir un composé de formule (V) sur un réactif d'oximation pour obtenir l'oxime de formule (IV) : 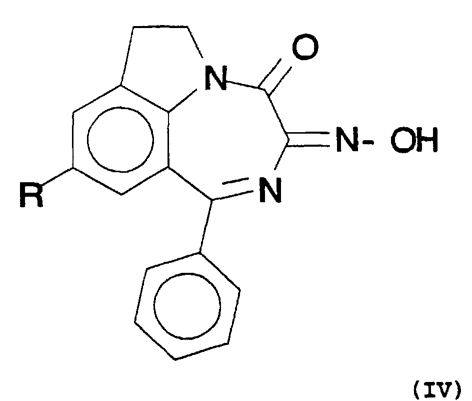 la seconde étape consistant à réduire l'oxime catalytiquement par de l'hydrogène en présence d'un catalyseur de réduction ou par réaction avec du zinc en présence d'acide acétique ou avec du chlorure stanneux en présence d'acide chlorhydrique, pour obtenir le dérivé aminé (II). Le schéma ci-après illustre le procédé de synthèse de (II):

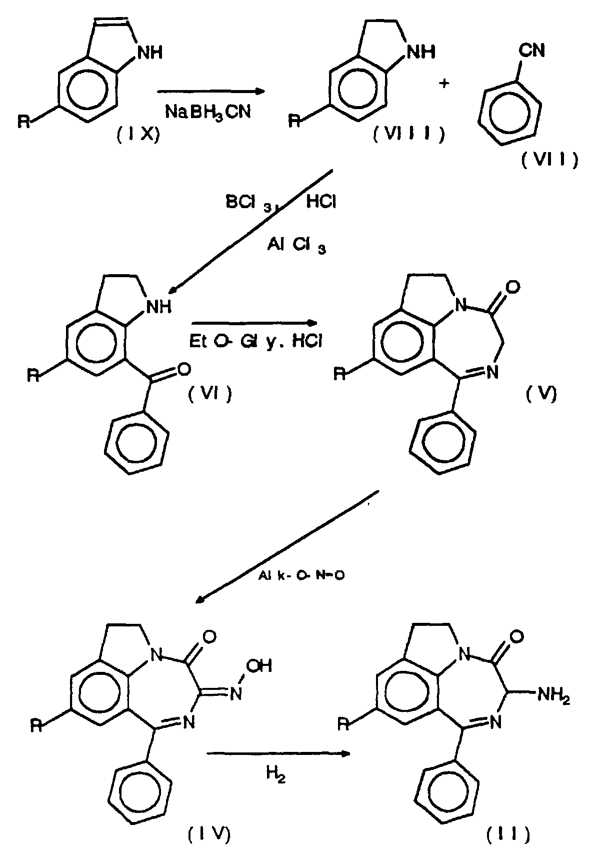

L'indole (IX) est réduit en l'indoline (VIII) correspondante, laquelle est condensée avec le benzonitrile (VII) en présence d'acide de Lewis pour donner, après hydrolyse, la benzophénone (VI).

La préparation, à partir de (VI) en présence de glycinate d'éthyle dans la pyridine, du produit de formule (V) est adaptée de la méthode décrite (méthode N) par Hester J.B. et al., 1970, J. Med. Chem. 13 : 827-835.

Entre autres possibilités, pour préparer un composé de formule (II) optiquement actif, on peut :
- condenser un composé (II) racémique avec un dérivé d'acide alpha aminé appartenant à la série D ou à la série L et dans lequel la fonction amine est protégée par un groupement facilement labile, de préférence le groupement tertiobutyloxycarbonyle.

Le composé obtenu est déprotégé par hydrolyse, de préférence en milieu acide en présence d'acide trifluoro-acétique, et le produit obtenu est séparé en ses diastéréoisomères par chromatographie ; on obtient les deux isomères de l'amine condensée avec l'acide aminé.

Par dégradation d'Edman, on revient ensuite aux deux énantiomères de l'amine (II) ; ou encore,
- dissoudre un composé (II) racémique dans une solution d'acide optiquement actif, comme par exemple un énantiomère des acides mandélique, dibenzoyltartrique, di-*p*-toluyitartrique, camphosulfonique, *p*-nitrobenzoylglutamique, ou tartrique, pour former deux sels diastéréoisomères, puis par différence de solubilité, en cristalliser sélectivement un dans un solvant approprié.

Les produits intermédiaires de formule (IV) et les produits de formule (II) sont des intermédiaires utiles pour la préparation des produits actifs suivant l'invention.

L'invention vise également un médicament pour lutter contre les maladies inflammatoires, allergiques, contre la bronchoconstriction, ou utile dans le traitement de l'asthme, caractérisé en ce qu'il comprend un diazépino-indole suivant l'invention, sous une forme pharmaceutique adaptée à l'affection à traiter.

### Partie expérimentale

### Partie chimique

Les exemples suivants illustrent, sans pour autant la limiter, la mise en oeuvre des procédés et les produits de l'invention. La pureté, l'identité et les caractéristiques physico chimiques des produits et des intermédiaires essentiels préparés sont déterminées, ainsi :
- la pureté est vérifiée par chromatographies sur couches minces de gel de silice (Merck 60 - F254) et le Rf observé est rapporté pour le solvant d'élution utilisé qui est, le plus fréquemment, identique à celui utilisé pour la purification chromatographique préparative des composés. Ces solvants sont identifiés par les sigles suivants :
   S.A : chorure de méthylène,
   S.A1 : chorure de méthylène - acétone , 97 - 3 (v/v),
   S.A2 : chorure de méthylène - acétone , 96 - 4 (v/v),
   S.A3 : chorure de méthylène - acétone , 95 - 5 (v/v),
   S.A4 : chorure de méthylène - acétone , 90-10 (v/v),
   S.A5 : chorure de méthylène - acétone , 88 -12 (v/v),
   S.A6 : chorure de méthylène - acétone , 85 - 15 (v/v),
   S.A7 : chorure de méthylène - acétate d'éthyle , 98 - 2 (v/v),
   S.A8 : chorure de méthylène - méthanol , 98 - 2 (v/v),
   S.A9 : chorure de méthylène - méthanol , 97 - 3 (v/v),
   S.A10 : chorure de méthylène - méthanol , 95 - 5 (v/v),
   S.B : acétate d'éthyle,
   S.B1 : acétate d'éthyle - cyclohexane, 70 - 30 (v/v),
   S.B2 : acétate d'éthyle - cyclohexane, 60 - 40 (v/v),
   S.B3 : acétate d'éthyle - méthanol , 97 - 3 (v/v),
   S.B4 : acétate d'éthyle - méthanol , 95 - 5 (v/v),
- l'identité de formule élémentaire des composés obtenus avec celle des structures visées est vérifiée par l'analyse des principaux éléments. Les résultats ne sont pas reportés mais indiqués conformes avec la structure proposée compte tenu d'éventuels solvats ou hydrates.
- l'identité des produits obtenus avec les structures proposées est vérifiée par leur spectre de résonance magnétique nucléaire du proton et par leur spectrographie infrarouge.

Les spectres R.M.N ¹H sont réalisés à 400 MHz sur un appareil de marque Brüker, les composés étant dissous dans le deutérochloroforme avec le tétraméthylsilane comme référence interne. La nature des signaux, leurs déplacements chimiques en ppm, le nombre de protons qu'ils représentent et leur capacité d'échange avec D₂O sont notés.

Les spectres infra rouge sont enregistrés en pastille de bromure de potassium sur un spectromètre Shimadzu IR-435.
- les caractéristiques physico chimiques relevées sont leur point de fusion, déterminés par la méthode du tube capillaire et dont les valeurs rapportées ne sont pas corrigées, leur pouvoir rotatoire, déterminés à la température ambiante voisine de 20°C sur un appareil Polartronic en cuve de 10 cm de long et dont les résultats permettent dans certains cas d'apprécier la pureté optique par calcul de l'excès énantiomérique (e.e.).

Dans un but de normalisation, la nomenclature chimique des produits exemplifiés est celle déterminée à l'aide du logiciel "Autonom" version 1.0 (Beilstein Institut - Ed. Springler) qui génère les nomenclatures systématiques des composés selon les règles de l'IUPAC. Egalement pour simplification, la nature du substituant R des produits exemplifiés n'est précisée que lorsqu'il est différent de H.

### Composés intermédiaires (II)

### Intermédiaire1.a:(3RS)-3-Amino-1-phényl-6,7-dihydro-3H-[1,4]diazépino[6,7,1-hi]indol-4-one (II-R,S).

Le composé est préparé selon le mode opératoire décrit à l'exemple 1 stades a) et b) de EP 0 340 064 A1.

### Intermédiaire 1.b : (3R)-3-Amino-1-phényl-6,7-dihydro-3H-[1,4]diazépino[6,7,1-hi]indol-4-one (II-R).

La préparation du composé est décrite à l'exemple 5 stades a) b) c) g) h) de la partie expérimentale de EP 0 340 064 A1. Toutefois, une méthode alternative qui consiste au dédoublement de l'intermédiaire racémique 1.a par la formation et la séparation de diastéréoisomères avec la N-acétyl-L-phényl-alanine est préférée.
- 74,0 g (267 mmol) de (3R,S)-3-Amino-1-phényl-6,7-dihydro-3H-[1,4]diazépino[6,7,1-hi]indol-4-one (int. 1.a) sont dissous dans 210 ml de n-propanol bouillant. Par ailleurs 44,1g (267 mmol) de N-acétyl-L-phényl-alanine sont dissous dans 140 ml de n-propanol bouillant. On mélange les deux solutions, laisse refroidir, amorce par quelques cristaux. Après trois jours de repos les cristaux sont filtrés et séchés. Poids : 50,0 g (e.e.= 77%). Le produit est recristallisé à deux reprises successives dans l'acétate d'éthyle bouillant. On obtient 39,0 g (e.e. = 97%). Les eaux mères de la première cristallisation sont évaporées, le résidu repris par de l'acétate d'éthyle bouillant. Après cristallisation, filtration et séchage on obtient 35,0 g de cristaux (e.e. = 50%), qui, après deux cristallisations successives dans l'acétate d'éthyle bouillant permettent d'obtenir 17,0 g (e.e. = 97%) de produit. Les deux jets réunis représentent 56,0 g (Rdt = 95 %) de sel de l'énantiomère 3R de l'amine avec la N-acétyl-L-phényl-alanine. F = 171°C. [α]_{D} = +132°(c = 1, méthanol).
- 42,4 g (96 mmol) du sel de l'amine 3R sont agités violemment en présence de 500ml d'acétate d'éthyle et de 500ml de soude normale. Après dissolution, la phase acétate d'éthyle est séparée, lavée par de l'eau saturée de chlorure de sodium, puis déshydratée et évaporée. On obtient 25,4 g d'amine intermédiaire 1.b. Rdt = 95%. F=79°C. [α]_{D} = 172° (c = 1, CH₂Cl₂).
R.M.N.¹H δ(ppm): 3,05-3,5 (m, 2H); 3,3 (s.large, 2H éch.); 3,9-4,0 (m, 1H); 4,6-4,7 (m, 1H); 7,05-7,6 (m, 9H).
I.R.:3350, 1670, 1600, 1560, 1420, 1380, 1340, 1290, 1240, 760, 730, 690^{cm-1}.

### Intermédiaire 2.a : (3R,S)-3-Amino-9-méthyl-1-phényl-6,7-dihvdro-3H-[1,4]diazépino[6,7,1-hi]indol-4-one (II - R,S ; R = CH₃).

### - Stade-1 : 5-méthyl-indoline.

A une solution de 20,0 g (152 mmol) de 5-méthyl-indole dans 300 ml d'acide acétique glacial, on ajoute en dessous de 20°C par petites quantités 28,74 g (457 mmol) de cyanoborohydrure de sodium. L'addition faiblement exothermique est effectuée en 3 heures et s'accompagne d'un léger dégagement d'hydrogène. On laisse 12 heures sous agitation en dessous de 20°C, puis ajoute 300 ml d'eau et ajuste le pH du milieu réactionnel entre 10 et 12 par addition de 500 ml d'une solution de soude à 30%. Le mélange est extrait deux fois par du dichlorométhane, puis la phase organique est lavée avec 100 ml d'eau. On évapore, le résidu est purifié par chromatographie rapide sur une colonne de silice, l'éluant utilisé étant un mélange de polarité croissante de méthanol dans du chlorure de méthylène. On obtient 15,3 g (Rdt = 75%) d'huile incolore qui se colore en marron au stockage (atmosphère d'azote et à l'abri de la lumière).
C.C.M.: S.A8; 0,39.
R.M.N.¹H δ(ppm): 2,2 (s, 3H); 2,95 (t, 2H); 3,4 (m, 3H dont 1 éch.); 6,5 (d, 1H); 6,8 (d, 1H); 6,95 (s, 1H).

### - Stade-2 : 7-benzoyl-5-méthyl-indoline.

On dissout 13,70 g (103 mmol) de 5-méthyl-indoline dans 360 ml de 1,2-dichloroéthane. On ajoute goutte à goutte à t< 5°C 13,24 g (113 mmol) de trichlorure de bore en solution molaire dans du dichlorométhane, 20,36 g (197 mmol) de benzonitrile puis 13,73 g (103 mmol) de trichlorure d'aluminium anhydre. Le mélange est chauffé 16 heures au reflux (t. masse = 82-84°C). Après refroidissement l'hydrolyse est réalisée par ajout de 103 ml d'acide chlorhydrique 4N et chauffage durant 20 minutes à 80°C. On laisse refroidir vers 20°C et extrait par du dichlorométhane. La phase aqueuse est réextraite par 100 ml de dichlorométhane. On lave les phases organiques réunies par une solution de soude, puis par une solution concentrée de chlorure de sodium, et sèche sur sulfate de sodium. Après filtration et évaporation on obtient 22,10 g de solide jaune Rdt = 91%. F = 84°C.
Analyse conforme pour C₁₆H₁₅NO - C.C.M.: S.A; 0,46
R.M.N.¹H δ(ppm): 2,2 (s, 3H); 3,05 (t, 2H); 3,75 (t, 2H); 6,9 (s.large, 1H éch.) ; 7,05 (s. large, 2H);7,5 (m, 3H), 7,65 (m, 2H).

### - Stade-3 : 9-méthyl-1-phényl-6,7-dihydro-3H-[1,4]diazépino[6,7,1-hi]indol-4-one.

Dans 140 ml de pyridine, on introduit 21,0 g (88 mmol) de 7-benzoyl-5-méthyl-indoline puis 43,2 g (31 mmol) de chlorhydrate de glycinate d'éthyle. On chauffe sous agitation à 110-115°C tout en distillant les fractions légères qui se forment. Après 12 heures on refroidit et ajoute 150 ml d'une solution de carbonate de sodium à 2,5% dans de l'eau et 150 ml de dichlorométhane. La phase aqueuse est séparée, extraite par 150 ml de dichlorométhane. Les phases organiques sont réunies et lavées à l'eau. Le solvant est évaporé puis le résidu purifié par chromatographie rapide sur une colonne de silice, l'éluant utilisé étant l'acétate d'éthyle. On obtient 22,0 g de produit purifié sous forme d'un solide beige-marron. Rdt = 80% - F = 132°C.
C.C.M.:S.B; 0,70.
R.M.N. 1H δ(ppm): 2,3 (s, 3H); 3,15 (t, 2H); 4,25 (t, 2H); 4,3 (t, 2H); 7,0 (s, 1H); 7,25 (s, 1H); 7,45 (m, 3H); 7,55 (m, 2H).

### - Stade-4 3-hydroxyimino-9-méthyl-1-phényl-6,7-dihydro-3H-[1,4]diazépino[6,7,1-hi] indol-4-one.

21,0 g (76mmol) du produit précédent sont dissous dans un mélange de 84 ml de tétrahydrofurane et de 168 ml de toluène. On refroidit et ajoute à une température inférieure à 0°C, 21,3 g (190 mmol) de tertio-butylate de potassium. L'addition est exothermique et la solution se colore en noir. Après 20 minutes d'agitation on ajoute en 10 minutes environ 9,35 g (80 mmol) de nitrite d'isoamyle. On maintient 10 minutes sous agitation en dessous de 0°C, puis ajoute 31,2 ml d'acide acétique glacial et 300 ml d'eau.

On filtre un insoluble et ajoute 200 ml de dichlorométhane. On décante et lave la phase aqueuse avec 200 ml de dichlorométhane. Les phases organiques sont réunies et lavées par 200 ml d'eau. Après évaporation du solvant le résidu est repris par 40 ml de méthanol. Le produit cristallisé est filtré, lavé par 20 ml de méthanol froid puis séché. On obtient 15,06 g d'un solide jaune. Rdt = 65% - F=247°C.
C.C.M.: S.B2; 0,38.
R.M.N. ¹H δ(ppm): 2,3 (s, 3H); 3,2 (t, 2H); 4,4 (t, 2H); 7,3 (m, 3H); 7,4-7,6 (m,3H); 7,9 (s.large, 1H).

### - Stade-5 :(3R,S)-3-Amino-9-méthyl-1-phényl-6,7-dihydro-3H-[1,4]diazépino[6,7,1-hi]indol-4-one.

On ajoute 1,32 g de ruthénium à 5 % sur charbon à une solution de 4,4 g (14,4mmol) du produit obtenu au stade précédent dans 150 ml de méthanol. On hydrogène sous une pression de 8 bars à 80°C pendant 6 heures, puis filtre et rince le catalyseur. Après évaporation le résidu est purifié par chromatographie rapide sur une colonne de silice, l'éluant utilisé étant un mélange d'acétate d'éthyle enrichi progressivement en méthanol. On obtient 2,87 g d'amine purifiée sous la forme d'un solide jaune-beige. Rdt = 68% - F=116°C. - C.C.M.:S.B4; 0,14.
R.M.N. ¹H δ(ppm): 2,3 (s, 3H); 2,4 (s.large, 2H éch.); 3,1 (m,1H); 3,3 (m, 1H); 3,95 (m, 1H); 4,65 (m,1H); 7-7,6 (m, 8H).

### Intermédiaire2.b: (3R)-3-Amino-9-méthyl-1-phényl-6,7-dihydro-3H-[1,4]diazépino[6,7,1-hi]indol-4-one. (II - R , R = CH₃)

19,95 g (68 mmol) de l'amine (R,S) 2.a sont dissous dans 200 ml d'acétonitrile au reflux. Par ailleurs, 26,45 g (68 mmol) d'acide di-para-toluoyl-tartrique sont dissous au reflux dans 260 ml d'acétonitrile. Les solutions chaudes sont mélangées, on laisse reposer 24 heures à la température du laboratoire. On filtre les cristaux blancs et les lave par 100 ml d'acétonitrile froid puis sèche. On détermine la pureté optique en faisant réagir 5 mg d'amine avec du 3-méthyl-isocyanate de phényle et en examinant le produit obtenu sur une colonne de chromatographie de type Pirckle en éluant par un mélange isopropanol/cyclohexane 50/50 (v/v). Les cristaux filtrés, poids 20,6 g (e.e. = 45), sont recristallisés à trois reprises successives dans de l'acétonitrile pour obtenir le produit purifié (e.e.=98). On obtient 12,0 g de produit. F=233°C. [α]_{D} = +177° (c = 1, méthanol).

Le sel précédent est mis en suspension dans 100 ml d'acétate d'éthyle. On ajoute, sous forte agitation une solution saturée de bicarbonate de sodium. Après quelques minutes on écarte la phase aqueuse. La phase organique est lavée à l'eau, séchée puis le solvant est évaporé à froid sous atmosphère d'azote. On obtient la base purifiée. F = 68°C.
[α]_{D} = +207° (c = 1, CH₂Cl₂).

### Intermédiaire 3.a : (3R,S)-3-Amino-9-méthoxy-1-phényl-6,7-dihydro-3H-[1,4]diazépino[6,7,1-hi]indol-4-one. (II - R,S; R = CH₃O)

Le composé est préparé à partir de 5-méthoxy-indole, en 5 stades, selon le mode opératoire décrit pour la préparation de l'intermédiaire 2.a.

### - Stade-1 : 5-Méthoxy-indoline.

Rdt = 83% - liquide jaune-clair qui se colore à la lumière. C.C.M.: S.B2; 0,38.
R.M.N.¹H δ(ppm): 3,0 (t, 2H); 3,41 (s, 1H éch.); 3,5 (t, 2H); 3,7 (s, 3H); 6,6 (s, 2H); 6,8 (s, 1H).

### - Stade-2 : 7-benzoyl-5-méthoxy-indoline.

Rdt = 38% -solide orange - F =123°C. C.C.M.: S.A7; 0,81
R.M.N.¹H δ(ppm): 3,05 (t, 2H); 3,65 (s, 3H); 3,75 (t, 2H); 6,75 (s.large, 2H dont 1H éch.); 6,95 (s.large,1H); 7,4-7,55 (m, 3H); 7,65 (m, 2H)

### - Stade-3 : 9-méthoxy-1-phényl-6,7-dihydro-3H-[1,4]diazépino[6,7,1-hi]indol-4-one.

Rdt = 82% - résine brune. C.C.M.: S.A6; 0,73.
R.M.N.¹H δ(ppm): 3,1 (t, 2H); 3,7 (s, 3H); 4,3 (t, 2H); 3,9 (s, 2H); 6,6 (s, 1H); 7,0 (s, 1H); 7,3-7,5 (m, 3H); 7,6 (d, 2H).

### - Stade-4 :3-hydroxyimino-9-méthoxy-1-phényl-6,7-dihydro-3H-[1,4]diazépino[6,7,1-hi] indol-4-one.

Rdt = 53% - solide jaune-orangé - F=205°C. ; C.C.M.: S.A5; 0,17.
R.M.N.¹H δ(ppm): 3,2 (t, 2H); 3,7 (s, 3H); 4,4 (t, 2H); 6,7 (t, 2H); 7,1 (s, 1H); 7,4-7,6 (m, 3H); 7,8 (d, 2H); 8,6 (s, 1H).

### - Stade-5 : (3R,S)-3-Amino-9-méthoxy-1-phényl-6,7-dihydro-3H-[1,4]diazépino[6,7,1-hi] indol-4-one.

Rdt = 67% - solide jaune-beige - F=84°C.; C.C.M.: S.B3; 0,24.
R.M.N.¹H δ(ppm): 3,2 (t, 2H); 3,7 (s, 3H); 4,4 (t, 2H); 5,3 (s, 1H); 6,7 (s, 1H); 7,1 (s, 1H); 7,4-7,8 (m, 5H); 2,1 et 8,5 (s. larges, 2H éch).

### Intermédiaire 3.b : (3R)-3-Amino-9-méthoxy-1-phényl-6,7-dihydro-3H-[1,4]diazépino[6,7,1-hi]indol-4-one (II - R; R = CH₃O)

10,0 g (32,3 mmol) de l'amine (R,S) 3.a sont dissous dans 100 ml d'acétonitrile au reflux. Par ailleurs, 12,47 g (32,3 mmol) d'acide di-para-toluoyl-tartrique sont dissous au reflux dans 100 ml d'acétonitrile. Les solutions chaudes sont mélangées puis abandonnées pour cristallisation par refroidissement à la température du laboratoire. Après une nuit de repos, on filtre les cristaux blancs et lave ceux-ci par 100 ml d'acétonitrile froid puis sèche. Ces cristaux (e.e. = 37 %) sont recristallisés à deux reprises successives dans de l'acétonitrile pour obtenir le produit purifié (e.e. =99,5 %). Cette purification est suivie par chromatographie sur une colonne optiquement active C₁₈ de type Pirckle, en éluant par un mélange 50/50 d'isopropanol et de n-hexane. On obtient 9,9 g de produit . Rdt = 44%. F=168°C.

Les 9,9 g du sel précédent sont mis en suspension dans 100 ml d'acétate d'éthyle. On ajoute sous forte agitation une solution saturée de bicarbonate de sodium, après quelques minutes on écarte la phase aqueuse. La phase organique est lavée par 50 ml d'eau, séchée, puis le solvant est évaporé à froid sous atmosphère d'azote. On obtient 4,1 g de base purifiée. Rdt = 95%
F = 84°C. [α]_{D} = +23° (c = 1,1, CH₂Cl₂).

### Exemples de l'invention (I)

Tel que décrit précédemment la préparation des composés (I) de l'invention met en oeuvre la réaction des 3-amino-1-phényl-6,7-dihydro-3H--[1,4]diazépino[6,7,1-hi]indol-4-ones, composés intermédiaires (II) avec des halogénures (III._{A}) selon le procédé A, des esters notamment pentafluorophényliques (III._{B}) selon le procédé B ou des acides carboxyliques (III._{C}) selon le procédé C. Les modes opératoires généraux de ces procédés sont présentés dans ce qui suit.

Procédé A : dans un réacteur protégé de l'humidité on dissout sous agitation 10,0 mmol d'une amine intermédiaire (II) dans 60 ml de chlorure de méthylène anhydre. A une température voisine de 20°C on ajoute alors 10,0 mmol d'halogénure d'acide (III_{A}) puis, goutte à goutte, 10,0 mmol de triéthylamine. La réaction est poursuivie sous agitation à la température ambiante comprise entre 15 et 25°C et son avancement est suivi par chromatographie sur couches minces. Lorsque la réaction est considérée terminée, on ajoute 120 ml de chlorure de méthylène au milieu réactionnel, le mélange est extrait successivement par 60 ml de solution HCl 1N, 60 ml de solution saturée de bicarbonate de sodium et finalement 60 ml d'eau. Après séchage le chlorure de méthylène est évaporé sous pression réduite, le résidu est purifié par chromatographie rapide sur une colonne de silice, le solvant d'élution étant un mélange de polarité croissante, constitué, par exemple, d'acétone dans le chlorure de méthylène. Les fractions d'élution déterminées contenir le composé pur sont réunies puis évaporées sous pression réduite. Le produit purifié résiduel est soumis aux déterminations structurales et de pureté précédemment décrites.

### - Procédé B :

stade 1 : dans 25 ml de dichlorométhane on dissout 10,0 mmol d'un acide intermédiaire (III_{C}) de formule A-COOH et 3,55 g (19,3 mmol) de pentafluorophénol. On ajoute ensuite 0,81 g (2,6 mmol) de para toluène sulfonate de para-diméthylamino-pyridinium et :
- soit 22,4 mmol de dicyclohexylcarbodiimide dans le procédé "B.a",
- soit 22,4 mmol de N-(3-diméthylaminopropyl)-N'-éthyl-carbodiimide dans le procédé "B.b".

Le mélange est agité durant 16 heures à la température du laboratoire voisine de 20°C puis l'insoluble est filtré. Le solvant est éliminé par distillation, le résidu est purifié par la technique de chromatographie rapide sur une colonne de silice en utilisant le plus couramment comme solvant d'élution un gradient d'acétone dans le chlorure de méthylène. Les fractions déterminées pures par C.C.M sont réunies, le solvant est évaporé et après analyse, l'ester intermédiaire (III_{B}) résiduel, sous forme de mousse amorphe, est utilisé tel quel dans le stade suivant.

stade 2 : 10,0 mmol de l'ester pentafluorophényle (III_{B}) préparé au stade précédent sont ajoutés à 10,0 mmol d'amine intermédiaire (II) dissous dans de l'acétate d'éthyle anhydre. Après 16 heures d'agitation à la température ambiante voisine de 20°C on filtre l'insoluble, évapore l'acétate d'éthyle sous vide puis purifie le résidu par la technique de chromatographie rapide sur une colonne de silice en utilisant le plus couramment comme solvant d'élution un gradient de méthanol dans le chlorure de méthylène. Les fractions déterminées pures par C.C.M sont réunies, le solvant est évaporé et le résidu purifié est identifié et analysé.

Procédé C : dans un réacteur protégé de l'humidité on dissout sous agitation 10,0 mmol d'une amine intermédiaire (II) dans 50,0 ml de chlorure de méthylène anhydre. A la température du laboratoire voisine de 20°C on ajoute alors 11,0 mmol d'un acide intermédiaire (III_{C}) de formule A-COOH puis 10,0 mmol (3,28 g) de "TOTU" (dénomination abrégée pour tétrafluoroborate de O-[(Ethoxycarbonyl)cyanométhylamino]-N,N,N',N'-tétraméthyluronium - fournisseur Fluka ref. 02580). Le mélange est refroidi à 0°C on ajoute alors 20,0 mmol (2,55 g) de N,N-diisopropyl-éthylamine après quoi le mélange est agité durant 12 heures à la température ambiante puis extrait successivement par 50 ml de solution HCl 1N, 50 ml d'une solution saturée de bicarbonate de sodium et finalement 50 ml d'eau.

Le solvant est évaporé sous vide et le résidu purifié par la technique de chromatographie rapide sur une colonne de silice en utilisant le plus couramment comme solvant d'élution un gradient de méthanol dans le chlorure de méthylène. Les fractions déterminées pures par C.C.M sont réunies, le solvant est évaporé et le résidu purifié est identifié et analysé.

### Exemple 1.A :(3R,S) 2-Chloro-4-trifluorométhyl-pyrimidine-5-carboxylic acid (4-oxo-1-phényl-3,4,6,7-tetrahydro-[1,4]diazépino[6,7,1-hi]indol-3-yl)-amide.

### [(I); A = 5-(2-Chloro-4-trifluorométhyl--pyrimidyl)]

Le composé est préparé selon le procédé A à partir de l'intermédiaire 1.a et du chlorure de l'acide 5-(2-Chloro-4-trifluorométhyl-)-pyrimidyl carboxylique.
Rdt = 72% - solide blanc - F=282°C (dec.).
Analyse conforme pour C₂₃H₁₅ClF₃N₅O₂ - C.C.M.: S.A4; 0,70.
R.M.N.¹H(DMSO) δ(ppm): 2,9-3,6 (m, 2H); 3,7-4,2 (m, 1H); 4,4-4,75 (m, 1H); 5,45 (d, 1H devient s. par éch.); 7,1-7,8 (m, 8H); 9,25 (s, 1H); 10,2 (d, 1H éch.)
I.R.: 3200, 1670, 1560, 1540, 1520, 1430, 1345, 1210, 1140, 800, 735, 700 cm⁻¹

### Exemple 1.B : (3R, Si Imidazo[1,2-a]pyridine-2-carboxylic acid N-(4-oxo-1-phényl-3,4,6,7-tetrahydro-[1,4]diazépino[6,7,1-hi]indol-3-yl)-amide.

### [(I); A = 2-Imidazo[1,2-a]pyridinyl]

Le composé est préparé à partir de l'intermédiaire 1.a et de l'acide Imidazo[1,2-a]pyridine-2-carboxylique selon une méthode dérivée du procédé C qui consiste à réaliser la condensation dans le tétrahydrofurane (THF) en présence d'hexafluorophosphate de bromo-tris-pyrrolidino-phosphonium ("PyBrop") et de triéthylamine. On dissout 4,50 g (16,23 mmole) de l'amine intermédiaire 1.a dans 150 ml de THF anhydre. On ajoute 3,20 g (16,3 mmole) d'acide Imidazo[1,2-a]pyridine-2-carboxylique et 4,95 g (6,82 ml, 49 mmole) de triéthylamine. Le mélange est refroidi par un bain de glace et on ajoute 9,13 g (18,6 mmole) de "PyBrop" dans 50 ml de THF. Après 16 heures sous agitation à la température du laboratoire l'insoluble est filtré et les solvants éliminés par distillation sous vide. Le résidu (12,2 g) est purifié par chromatographie sur colonne de silice en éluant par l'acétate d'éthyle contenant 5 % d'acétone. Les fractions déterminées par C.C.M contenir le produit purifié sont réunies et le solvant évaporé. On obtient 5,1 g de produit pur sous forme amorphe. Rdt = 71% - F = 260°C
Analyse conforme pour C₂₅H₁₉FN₅O₂, H₂O - C.C.M.: S.A6; 0,27
R.M.N.¹H δ(ppm): 2,80-3,55 (m,2H); 3,70-4,10 (m,1H); 4,30-4,75 (m,1H); 5,50-5,68 (d, 1H s. par éch.); 6,65-7,70 (m,11H); 8,00-8,15 (m,2H); 8,85-8,95 (d, 1H éch.)
I.R.: 3100, 1725, 1640, 1520, 1390, 1275, 1020, 820, 800, 750 cm⁻¹

### Exemple 2.A : (3R) 2-Fluoro-N-(4-oxo-1-phényl-3,4,6,7-tétrahydro-[1,4]diazépino[6,7,1-hi]indol-3-yl)-benzamide

### [(I); A = 2-fluorophényl]

Composé préparé selon le procédé A à partir de l'intermédiaire 1.b et du chlorure de 2-fluorobenzoyle.
Rdt = 50,5% - solide amorphe - F=192°C - [α]_{D} = +51° (c = 1, CH₂Cl₂)
Analyse conforme pour C₂₄H₁₈FN₃O₂ - C.C.M.: S.A3; 0,43
R.M.N.¹H δ(ppm): 3,10-3,20 (m, 1H); 3,30-3,45 (m, 1H); 3,95-4,05 (m, 1H); 4,65-4,75 (m, 1H); 5,65 (d, 1H); 7,10-7,60 (m, 11H dont 1H éch.); 8,15 (m, 1H), 8,50-8,65 (m, 1H).
I.R.: 3300, 1640, 1490, 1430, 1380, 1340, 1230, 1160, 725, 690 cm⁻¹

### Exemple 2.B : (3R) 3-Fluoro-N-(4-oxo-1-phényl-3,4,6,7-tétrahydro-[1,4]diazépino[6,7,1-hi]indol-3-yl)-benzamide

### [(I); A = 3-fluorophényl]

Composé préparé selon le procédé A à partir de l'intermédiaire 1.b et du chlorure de 3-fluorobenzoyle.

Rdt = 78% - solide amorphe - F=244°C - [α]_{D} = +48° (c = 1, CH₂Cl₂)
Analyse conforme pour C₂₄H₁₈FN₃O₂, 0,5H₂O - C.C.M.: S.A3; 0,43.
R.M.N.1H δ(ppm): 3,15 (m, 1H); 3,35 (m, 1H); 4,00 (m, 1H); 4,65 (m, 1H); 5,60 (d, 1H); 7,10-7,60 (m, 10H); 7,65-7,80 (m, 2H); 8,00 (d, 1H éch.).
I.R.: 3250, 1670, 1620, 1580, 1520, 1430, 1380, 1340, 1280, 1240, 1220, 1140, 790, 670 cm⁻¹

### Exemple 2.C : (3R) 4-Fluoro-N-(4-oxo-1-phényl-3,4,6,7-tétrahydro-[1,4]diazépino[6,7,1-hi]indol-3-yl)-benzamide

### [(I); A = 4-fluorophényl]

Composé préparé selon le procédé A à partir de l'intermédiaire 1.b et du chlorure de 4-fluorobenzoyle.
Rdt = 50,4% - solide amorphe - F=228°C - [α]_{D} = +48° (c = 1, CH₂Cl₂)
Analyse conforme pour C₂₄H₁₈FN₃O₂, 0,25H₂O - C.C.M.: S.A3; 0,52.
R.M.N.1H δ(ppm): 3,15 (m, 1H); 3,35 (m, 1H); 3,95 (m,1H); 4,65 (m, 1H); 5,60 (d, 1H); 7,10-7,60 (m, 10H dont 1H éch.); 8,00 (m, 1H).
I.R.:3400, 1640, 1590, 1490, 1440, 1420, 1380, 1340, 1230, 1160, 1050, 800, 760, 690, 660 cm⁻¹

### Exemple 2.D : (3R) 2-Chloro-N-(4-oxo-1-phényl-3,4,6,7-tétrahydro-[1,4]diazépino[6,7,1-hi]indol-3-yl)-benzamide

### [(I); A = 2-chlorophényl]

Composé préparé selon le procédé A à partir de l'intermédiaire 1.b et du chlorure de 2-chlorobenzoyle.
Rdt = 66% - solide amorphe - F=121°C - [α]_{D} = +82° (c = 1, CH₂Cl₂)
Analyse conforme pour C₂₄H₁₈FN₃O₂ , 0,1CH₂Cl₂ - C.C.M.: S.A3; 0,57.
R.M.N.1H δ(ppm): 3,15 (m, 1H); 3,35 (m, 1H); 4,00 (m, 1H); 4,65 (m, 1H); 5,60 (d, 1H); 7,10 (m, 1H); 7,20-7,60 (m, 10H); 7,80 (m, 1H); 8,00 (d, 1H éch.).
I.R.:3300, 1650, 1590, 1490, 1430, 1380, 1220, 1160, 1040, 750, 730, 690 cm⁻¹

### Exemple 2.E : (3R) 3-Chloro-N-(4-oxo-1-phényl-3,4,6,7-tétrahydro-[1,4]diazépino[6,7,1-hi]indol-3-yl)-benzamide

### [(I); A = 3-chlorophényl]

Composé préparé selon le procédé A à partir de l'intermédiaire 1.b et du chlorure de 3-chlorobenzoyle.
Rdt = 85% - solide blanc - F=230°C (dec.) - [α]_{D} = +34° (c = 1, CH₂Cl₂)
Analyse conforme pour C₂₄H₁₈ClN₃O₂ - C.C.M.: S.A3; 0,49.
R.M.N.1H δ(ppm): 3,15 (m, 1H); 3,35 (m, 1H); 4,0 (m, 1H); 4,67 (m, 1H); 5,65 (d, 1H s. par éch.); 7,15 (m, 1H); 7,25 (m, 1H); 7,3-7,6 (m, 8H); 7,85 (m, 1H); 8,0 (m, 1H); 8,15 (d, 1H éch.).
I.R.: 3250(large), 3050, 1680, 1650, 1505, 1440, 1275, 1240, 725, 690 cm⁻¹

### Exemple 2.F : (3R) 2-Iodo-N-(4-oxo-1-phényl-3,4,6,7-tétrahydro-[1,4]diazépino[6,7,1-hi]indol-3-yl)-benzamide

### [(I); A = 2-iodophényl]

Composé préparé selon le procédé A à partir de l'intermédiaire 1.b et du chlorure de 2-iodobenzoyle.
Rdt = 69% - solide amorphe - F=123°C - [α]_{D} = +81° (c = 1, CH₂Cl₂)
Analyse conforme pour C₂₄H₁₈IN₃O₂ - C.C.M.: S.A3; 0,55.
R.M.N.¹H δ(ppm): 3,15 (m, 1H); 3,40 (m, 1H); 4,0 (m, 1H); 4,67 (m, 1H); 5,65 (d, 1H s. par éch.); 7,12 (m, 2H); 7,25 (m, 1H); 7,3-7,5 (m, 5H); 7,58 (m, 2H); 7,70 (m, 2H dont 1 éch.); 7,92 (d, 1H).
I.R.:3400, 3260, 1650, 1490, 1440, 1385, 1160, 725, 690 cm⁻¹

### Exemple 2.G : (3R) 3-Chloro-4-fluoro-N-(4-oxo-1-phényl-3,4,6,7-tétrahydro-[1,4]diazépino[6,7,1-hi]indol-3-yl)-benzamide

### [(I); A = 3-chloro-4-fluorophényl]

Composé préparé selon le procédé C à partir de l'intermédiaire 1.b et d'acide 3-chloro-4-fluoro-benzoïque.
Rdt = 97% - solide amorphe - F=148°C - [α]_{D} = +43° (c = 1, CH₂Cl₂)
Analyse conforme pour C₂₄H₁₇ClFN₃O₂ - C.C.M.: S.A3; 0,70.
R.M.N.¹H δ(ppm): 3,15 (m, 1H); 3,35 (m, 1H); 4,00 (m, 1H); 4,65 (m, 1H); 5,60 (d, 1H); 7,10-7,60 (m, 9H dont 1H éch.); 7,85 (m, 1H); 8,05 (m, 2H).
I.R.: 3300, 3050, 1650, 1480, 1380, 1250, 1170, 1050, 750, 730, 690 cm¹

### Exemple 2.H : (3R) 3,4-dichloro-N-(4-oxo-1-phényl-3,4,6,7-tétrahydro-[1,4]diazépino[6,7,1-hi]indol-3-yl)-benzamide.

### [(I); A = 3,4-dichlorophényl]

Préparé selon le procédé A à partir de l'intermédiaire 1.b et du chlorure de 3,4-dichlorobenzoyle.
Rdt = 85,4% - solide amorphe - F=163°C - [α]_{D} = +42° (c = 1, CH₂Cl₂)
Analyse conforme pour C₂₄H₁₇Cl₂N₃O₂ - C.C.M.: S.A3; 0,76.
R.M.N.¹H δ(ppm): 3,15 (m, 1H); 3,35 (m, 1H); 4,00 (m, 1H); 4,65 (m, 1H); 5,60 (d, 1H); 7,10-7,60 (m, 9H dont 1H éch.); 7,85 (m, 1H); 8,05 (m, 2H)
I.R.: 3300, 3050, 1640, 1500, 1440, 1380, 1280, 1230, 1130, 1020, 750, 730, 690 cm⁻¹

### Exemple 2.1 : (3R) 2-Méthyl-N-(4-oxo-1-phényl-3,4,6,7-tétrahydro-[1,4]diazépino[6,7,1-hi]indol-3-yl)-benzamide

### [(I); A = 2-méthylphényl]

Préparé selon le procédé A à partir de l'intermédiaire 1.b et du chlorure de 2-méthylbenzoyle.
Rdt = 33% - solide blanc - F = 154°C - [α]_{D} = +78° (c = 1, CH₂Cl₂)
Analyse conforme pour C₂₅H₂₁N₃O₃ - C.C.M.: S.A3; 0,34.
R.M.N.¹H δ(ppm): 2,6 (s, 3H); 3,15 (m, 1H); 3,38 (m, 1H); 3,48 (m, 1H); 4,68 (m, 1H); 5,66 (d, 1H s. par éch.); 7,12 (m, 1H); 7,25 (m, 3H); 7,3-7,5 (m, 5H); 7,56 (m, 2H); 7,68 (m, 2H ,1H éch.).
I.R.: 3300, 3000, 1650, 1470, 1440, 1380, 1250, 1160, 725, 690 cm⁻¹

### Exemple2.J: (3R)2-Méthoxy-N-(4-oxo-1-phényl-3,4,6,7-tétrahydro-[1,4]diazépino[6,7,1-hi]indol-3-yl)-benzamide

### [(I); A = 2-méthoxyphényl]

Composé préparé selon le procédé A à partir de l'intermédiaire 1.b et du chlorure de 2-méthoxybenzoyle.
Rdt = 72% - solide blanc -F= 228°C - [α]_{D} = +34° (c = 1, CH₂Cl₂)
Analyse conforme pour C₂₅H₂₁N₃O₃,(0.25H₂O). - C.C.M.: S.A3; 0,53.
R.M.N.¹H δ(ppm): 3,15 (m, 1H); 3,37 (m, 1H); 4,0 (m, 1H); 4,08 (s, 3H); 4,67 (m, 1H); 5,70 (d, 1H s. par éch.); 7,0-7,15 (m, 3H); 7,25 (m, 1H); 7,38 (m, 2H); 7,45 (m, 3H); 7,55 (m, 2H); 8,25 (m, 1H); 9,85 (m, 1H éch.).
I.R.: 3350, 2900, 1670, 1640, 1590, 1500, 1470, 1380, 1280, 1240, 1170, 1010, 750, 730, 690 cm⁻¹

### Exemple 2.K : (3R) 3-Méthoxy-N-(4-oxo-1-phényl-3,4,6,7-tétrahydro-[1,4]diazépino[6,7,1-hi]indol-3-yl)-benzamide

### [(I); A = 3-méthoxyphényl]

Composé préparé selon le procédé A à partir de l'intermédiaire 1.b et du chlorure de 3-méthoxybenzoyle.
Rdt = 74% - solide blanc -F= 181°C - [α]_{D} = +48° (c = 1, CH₂Cl₂)
Analyse conforme pour C₂₅H₂₁N₃O₃ - C.C.M.: S.A3; 0,51.
R.M.N.¹H δ(ppm): 3,15 (m, 1H); 3,35 (m, 1H); 3,85 (s, 3H); 4,0 (m, 1H); 4,65 (m, 1H); 5,65 (d, 1H s. par éch.); 7,10 (m, 2H); 7,25 (m, 1H); 7,35-7,6 (m, 9H); 7,97 (m, 1H éch.)
I.R.: 3400, 2900, 1650, 1600, 1480, 1500, 1475, 1440, 1380, 1275, 1240, 1030, 790, 750, 720, 695 cm⁻¹

### Exemple 2.L : (3R) 4-Méthoxy-N-(4-oxo-1-phényl-3,4,6,7-tétrahydro-[1,4]diazépino[6,7,1-hi]indol-3-yl)-benzamide

### [(I); A = 4-méthoxyphényl]

Composé préparé selon le procédé A à partir de l'intermédiaire 1.b et du chlorure de 4-méthoxybenzoyle.
Rdt = 67% - solide blanc -F= 221°C (dec.) -[α]_{D} = +51° (c = 1, CH₂Cl₂)
Analyse conforme pour C₂₅H₂₁N₃O₃ - C.C.M.: S.A3; 0,49.
R.M.N.¹H δ(ppm): 3,13 (m, 1H); 3,35 (m, 1H); 3,85 (s, 3H); 4,0 (m, 1H); 4,65 (m, 1H); 5,65 (d, 1H s. par éch.); 6,95 (d, 2H); 7,10 (t, 1H); 7,25 (t, 1H); 7,38 (m, 2H); 7,45 (m, 2H); 7,55 (m, 2H); 7,95 (m, 3H dont 1 éch.)
I.R.: 3350, 1680, 1650, 1600, 1480, 1390, 1250, 1200, 1030, 840, 760, 725, 695 cm⁻¹

### Exemple 2.M : (3R) 3,4,5-Triméthoxy-N-(4-oxo-1-phényl-3,4,6,7-tétrahydro-[1,4]diazépino[6,7,1-hi]indol-3-yl)-benzamide

### [(I); A = 3,4,5-triméthoxyphényl]

Composé préparé selon le procédé C à partir de l'intermédiaire 1.b et de l'acide 3,4,5-triméthoxybenzoïque.
Rdt = 81,4% - solide amorphe - F= 221°C (déc.) [α]_{D} = +54° (c = 1, CH₂Cl₂)
Analyse conforme pour C₂₇H₂₅N₃O₅ - C.C.M.: S.A4; 0,76.
R.M.N.¹H δ(ppm): 3,15 (m, 1H); 3,35 (m, 1H); 3,90 (d, 9H); 4,00 (m, 1H); 4,65 (m, 1H); 5,60 (d, 1H); 7,10 (m, 1H); 7,15-7,30 (m, 3H); 7,30-7,60 (m, 6H); 7,90 (d, 1H)
I.R.: 3300, 2900, 1640, 1520, 1470, 1300, 1230, 1170, 1120, 1000, 750, 730, 690 cm⁻¹

### Exemple 2.N : (3R) 2-Méthoxy-5-chloro-N-(4-oxo-1-phényl-3,4,6,7-tétrahydro-[1,4]diazépino[6,7,1-hi]indol-3-yl)-benzamide

### [(I); A = 2-méthoxy-5-chlorophényl]

Composé préparé selon le procédé C à partir de l'intermédiaire 1.b et de l'acide 2-méthoxy-5-chlorobenzoïque.
Rdt = 90,1% - solide amorphe - F= 234°C - [α]_{D} = nul (c = 1, CH₂Cl₂)
Analyse conforme pour C₂₅H₁₇ClN₃O₃ - C.C.M.: S.A3; 0,64.
R.M.N.¹H δ(ppm): 3,15 (m, 1H); 3,35 (m, 1H); 4,00 (m, 1H); 4,10 (s, 3H); 4,70 ( m, 1H); 5,6( (d, 1H); 7,00 (d, 1H); 7,10 (t, 1H); 7,20-7,50 (m, 6H); 7,55 (d, 2H); 8,2 (m, 1H); 9,75 (d, 1H éch.)
I.R.: 3350, 1650, 1590, 1470, 1380, 1260, 1240, 1180, 1010, 730, 690, 640 cm⁻¹

### Exemple 2.O : (3R) 4-acétamido-N-(4-oxo-1-phényl-3,4,6,7-tétrahydro-[1,4]diazépino[6,7,1-hi]indol-3-yl)-benzamide

### [(I); A = 4-acétamidophényl]

Composé préparé selon le procédé B.a à partir de l'intermédiaire 1.b et de l'ester pentafluorophénylique intermédiaire obtenu avec l'acide 4-acétamido-benzoïque.
Rdt = 32% - produit amorphe - F = 266°C - [α]_{D} = +43° (c = 1, CH₂Cl₂)
Analyse conforme pour C₁₆H₂₂N₄O₃ - C.C.M.: S.B; 0,20.
R.M.N.¹H δ(ppm): 2,10 (s, 3H); 3,15 (m, 1H); 3,35 (m, 1H); 4,00 (m, 1H); 4,65 (m, 1H); 5,60 (d, 1H); 7,10-7,60 (m, 8H); 7,65 (d, 2H); 7,85 (d, 2H); 7,95 (d, 1H éch.); 8,35 (s.large, 1H éch.)
I.R.: 3300, 1690, 1740, 1600, 1510, 1440, 1390, 1310, 1260, 1180, 1120, 1020, 860, 760, 730, 700 cm⁻¹

### Exemple 2.P : (3R) Pyridine-2-carboxylic acid (4-oxo-1-phényl-3,4,6,7-tetrahydro-[1,4]diazépino[6,7,1-hi]indol-3-yl)-amide

### [(I); A = 2-pyridyl]

Composé préparé selon le procédé C à partir de l'intermédiaire 1.b et de l'acide pyridine-2-carboxylique.
Rdt = 86,0% - solide amorphe - F= 208°C - [α]_{D} = +57° (c = 1, CH₂Cl₂)
Analyse conforme pour C₂₃H₁₈N₄O₂, 0,2 CH₂Cl₂, 0,1 H₂O - C.C.M.: S.A3; 0,67. R.M.N.¹H δ(ppm): 3,15 (m, 1H); 3,35 (m, 1H); 4,00 (m, 1H); 4,65 (m, 1H); 5,65 (d, 1H); 7,10 (m, 1H); 7,20-7,60 (m, 8H); 7,85 (m, 1H); 8,20 (d, 1H); 8,65 (d, 1H); 9,70 (d, 1H éch.)
I.R.: 3300, 1660, 1490, 1440, 1380, 1240, 1160, 750, 690 cm⁻¹

### Exemple 2.Q:(3R)N-(4-oxo-1-phényl-3,4,6,7-tétrahydro-[1,4]diazépino[6,7,1-hi]indol-3-yl)-isonicotinamide

### [(I); A = 4-pyridyl]

Composé préparé selon le procédé B.a à partir de l'intermédiaire 1.b et de l'ester pentafluorophénylique intermédiaire obtenu avec l'acide isonicotinique.
Rdt = 83% - produit amorphe - F = 234°C - [α]_{D} = +23° (c = 1, CH₂Cl₂)
Analyse conforme pour C₂₅H₂₀ClN₃O₃ - C.C.M.: S.A10; 0,60.
R.M.N.¹H δ(ppm): 3,10 (m, 1H); 3,30 (m, 1H); 3,90 (m, 1H); 4,60(m,1H); 5,60 (d, 1H); 7,05-7,50 ( m, 8H dont 1H éch.); 7,75 (m, 2H); 8,50 (m, 1H); 8,70 (m, 2H)
I.R.: 3300, 1640, 1470, 1430, 1380, 1270, 1160, 1110, 1040, 750, 720, 690 cm⁻¹

### Exemple 2.R : (3R) 1H-Indole-2-carboxylic acid (4-oxo-1-phényl-3,4,6,7-tetrahydro-[1,4]diazépino[6,7,1-hi]indol-3-yl)-amide.

### [(I); A = 2-1H-indolyl]

Composé préparé selon le procédé A à partir de l'intermédiaire 1.b et du chlorure d'acide 1H-indole-2-carboxylique.
Rdt = 81% - solide blanc -F= 196°C - [α]_{D} = +74° (c = 1, CH₂Cl₂)
Analyse conforme pour C₂₆H₁₉N₄O₂, 0,5 C₄H₈O₂. - C.C.M.: S.A3; 0,62.
R.M.N.¹H δ(ppm): 2,8-3,6 (m, 2H); 3,7-4,25 (m, 1H); 4,4-4,7 (1H); 5,55-5,65 (d, 1H s. par éch.); 6,9-7,7 (m, 13H); 7,9-8,1 (d, 1H éch.); 10,05 (s, 1H éch.)
I.R.: 3250, 1685, 1630, 1530, 1440, 1385, 1340, 1235, 740, 690 cm⁻¹

### Exemple 2.S : (3R) Quinoline-3-carboxylic acid (4-oxo-1-phényl-3,4,6,7-tetrahydro-[1,4]diazépino[6,7,1-hi]indol-3-yl)-amide.

### [(I); A = 3-quinolyl]

Préparé selon le procédé A à partir de l'intermédiaire 1.b et du chlorure de l'acide quinoline-3-carboxylique.
Rdt = 65% - solide blanc -F= 150°C - [α]_{D} = +152° (c = 1, CH₂Cl₂)
Analyse conforme pour C₂₇H₂₀N₄O₂, 0,4 H₂O - C.C.M.: S.A4; 0,22.
R.M.N.¹H δ(ppm): 2,70-3,6 (m, 2H); 3,6-4,15 (m, 1H); 4,3-4,8 (1H); 5,5-5,7 (d, 1H s. par éch.); 6,8-8,3 (m, 13 H); 8,60-8,8 (m, 1H); 9,2-9,4 (m, 1H)
I.R.:3300, 1680, 1660, 1510, 1445, 1285, 840, 785, 695 cm⁻¹

### Exemple 2.T : (3R) Quinoline-6-carboxylic acid (4-oxo-1-phényl-3,4,6,7-tetrahydro-[1,4]diazépino[6,7,1-hi]indol-3-yl)-amide.

### [(I); A = 6-quinolyl]

Préparé selon le procédé A à partir de l'intermédiaire 1.b et du chlorure de l'acide quinoline-6-carboxylique.
Rdt = 57% - solide blanc -F= 182°C - [α]_{D} = +59° (c = 1, CH₂Cl₂)
Analyse conforme pour C₂₇H₂₀N₄O₂, 0,75 H₂O - C.C.M.: S.A4; 0,40.
R.M.N.¹H δ(ppm): 2,80-3,60 (m, 2H); 3,70-4,30 (m, 1H); 4,50-4,90 (m, 1H); 5,75 (d, 1H s. par éch.); 6,90-7,70 (m, 10H); 8,00-8,40 (m, 4H dont 1H éch.); 8,55 (m, 1H); 9,00 (m, 1H)
I.R.: 3300, 1680, 1650, 1510, 1490, 1440, 1280, 780, 730, 695 cm⁻¹

### Exemple 2.U : (3R) 2-Méthyl-quinoline-4-carboxylic acid (4-oxo-1-phényl-3,4,6,7-tetrahydro-[1,4]diazépino[6,7,1-hi]indol-3-yl)-amide.

### [(I); A = 4-(2-méthyl-quinolyl)]

Composé préparé selon le procédé B.a à partir de l'intermédiaire 1.b et de l'ester pentafluorophénylique intermédiaire obtenu avec l'acide2-Méthyl-quinoline-4-carboxylique
Rdt = 94% - produit amorphe - F = 280°C (déc.)
Analyse conforme pour C₂₈H₂₂N₄O₂, 0,1 CH₂Cl₂ - C.C.M.: S.A3; 0,58.
R.M.N.¹H δ(ppm): 2,70 (s, 3H); 3,20 (m, 1H); 3,45 (m, 1H); 4,00 (m, 1H); 4,70 (m, 1H); 5,80 (d, 1H); 7,10-7,75 (m, 11H); 8,15 (d, 1H); 8,20 (d, 1H éch.); 8,85 (s, 1H)
I.R.: 3300, 1650, 1600, 1520, 1450, 1390, 1340, 1240, 1160, 760,700 cm⁻¹

### Exemple 2.V : (3R) Isoquinoline-3-carboxylic acid (4-oxo-1-phényl-3,4,6,7-tetrahydro-[1,4]diazépino[6,7,1-hi]indol-3-yl)-amide

### [(I); A = 3-isoquinolyl]

Composé préparé selon le procédé B.b à partir de l'intermédiaire 1.b et de l'ester pentafluorophénylique intermédiaire obtenu avec l'acide 3-Méthyl-quinoline-4-carboxylique en présence de N-(3-diméthylaminopropyl)-N'-éthylcarbodiimide. Rdt = 84% - produit amorphe - F = 250°C - [α] _{D} = +10° (c =1, CH₂Cl₂).
Analyse conforme pour C₂₇H₂₀N₄O₂, 0,3 H₂O, 0,15 CH₂Cl₂. - C.C.M.: S.A1; 0,25.
R.M.N.:¹H δ(ppm): 3,12 (m, 1H); 3,38 (m, 1H); 3,75 (s, 3H); 4,0 (m, 1H); 4,63 (m, 1H); 5,72 (d, 1H s. par ech.); 7,12 (m, 1H); 7,28 (m, 1H); 7,35 (m, 2H); 7,47 (m, 2H); 7,59 (m, 2H); 7,72 (m, 2H); 8,0 (d, 1H); 8,1 (d, 1H); 8,65 (s, 1H); 9,28 (s, 1H); 9,86 (d, 1H éch.).
I.R.: 3400, 1670, 1600, 1485, 1450, 1260, 900, 790, 785, 755, 730, 700, 670 cm⁻¹
- Chlorhydrate préparé à partir de la base, dans le propanol chlorhydrique. Les cristaux obtenus sont rincés par de l'éther et séchés sous vide.
   F=225°C. [α]_{D} = -416° (c = 1, CH₂Cl₂). - C.C.M.: S.A3; 0,35.

### Exemple 2.W: Fluorosulfonate de (3R) N-Méthyl-isoquinolinium-3-carboxylic acid (4-oxo-1-phényl-3,4,6,7-tetrahydro-[1,4]diazépino[6,7,1-hi]indol-3-yl)-amide

### [(I); A = N-méthyl-3-Isoquinoléinium]

1,0g (2,31 mmol) de produit de l'exemple 2.V sont dissous dans 12 ml de dichlorométhane, la solution est refroidie à 0°C et on y ajoute 0,184g (2,31 mmol) de fluorosulfonate de méthyle. Après une heure à 0°C on laisse revenir à la température ambiante puis évapore sous pression réduite. Le résidu est purifié par chromatographie rapide sur une colonne de silice, le solvant d'élution étant un mélange de polarité croissante d'acétone dans du chlorure de méthylène. On obtient 1,1g du composé sous forme d'un solide blanc. F=228°C. Rdt = 87% [α]_{D} = -408° (c=1, CH₂ Cl₂)
Analyse conforme pour C₂₈H₂₃FN₄O₅S, 0,5 H₂O - C.C.M.: S.A10; 0,10.
R.M.N.¹H δ(ppm): 3,35 (m, 1H); 3,45 (m, 1H); 3,52 (s, 3H); 4,5 (m, 1H); 4,78 (m, 1H); 5,75 (d, 1H s. par éch.); 7,0 (m, 1H); 7,25 (m, 1H); 7,45 (m, 1H); 7,65 (m, 2H); 7,7-7,9 (m, 5H); 8,02 (m, 1H); 8,12 (s, 1H); 8,65 (s, 1H); 9,25 (s, 1H); 10,5 (d, 1H éch.)
I.R.: 3450, 3350, 1680, 1600, 1560, 1480, 1430, 1270, 1070, 770, 700, 580 cm⁻¹

### Exemple 2.X : Imidazo[1,2-a]pyrimidine-2-carboxylic acid (4-oxo-1-phényl-3,4,6,7-tetrahydro-[1,4]diazépino[6,7,1-hi]indol-3-yl)-amide.

### [(I); A = Imidazo[1,2-a]pyrimidinyl]

Composé préparé selon le procédé B.a à partir de l'intermédiaire 1.b et de l'ester pentafluorophénylique intermédiaire obtenu avec l'acide Imidazo[1,2-a]pyrimidine-2--carboxylique.
Rdt = 57% - produit amorphe - F = 270°C (déc.) - [α]_{D} = -21° (c=1, CH₂Cl₂)
Analyse conforme pour C₂₄H₁₈N₆O₂, 0,5 C₃H₆O, 0,2 CH₂Cl₂ - C.C.M.: S.A9; 0,13.
R.M.N.(D.M.S.O.)¹H δ(ppm): 2,90-3,5 (m, 2H); 3,5-4,20 (m, 1H); 4,2-4,7 (m, 1H); 5,40-5,6 (d, 1H s. par éch.); 7,0-7,7 (m, 9H); 7,6-7,8 (m, 1H); 8,0 (s, 1H); 8,6-8,75 (m, 1H); 8,75-8,95 (d, 1H éch.).
I.R.:3400(large), 3200, 1685, 1645, 1530, 1440, 1280, 1220, 1155, 730, 700 cm⁻¹

### Exemple 2.Y : (3R)4,7-Diméthyl-pyrazolo[5,1-c][1,2,4]triazine-3-carboxylic acid(4-oxo-1-phényl-3,4,6,7-tetrahydro-[1,4]diazépino[6,7,1-hi]indol-3-yl)-amide

### [(I); A = 3-(4,7-Diméthyl-pyrazolo[5,1-c][1,2,4]triazinyl)]

Composé préparé selon le procédé B.a à partir de l'intermédiaire 1.b et de l'ester pentafluorophénylique intermédiaire obtenu avec l'acide 4,7-Diméthyl-pyrazolo[5,1-c][1,2,4]triazine-3-carboxylique.
Rdt = 46%. Poudre blanche, F=260°C - [α]_{D} = +21° (c = 1, CH₂Cl₂)
Analyse conforme pour C₂₅H₂₁N₇O₂, 0,75 H₂O - C.C.M.: S.A3; 0,20.
R.M.N.¹H δ(ppm): 2,65 (s, 3H); 3,15 (m, 1H); 3,30 (s, 3H) 3,40 (m, 1H); 4,00 (m, 1H); 4,70 (m, 1H); 5,70 (d, 1H); 7,10-7,60 (m, 9H); 9,90 (d, 1H éch.)
I.R.: 3350, 1660, 1600, 1560, 1470, 1390, 1370, 1300, 1230, 1170, 800, 730, 690, 640 cm⁻¹

### Exemple 3 : (3R,S) Quinoline-3-carboxylic acid (9-méthyl-4-oxo-1-phényl-3,4,6,7-tetrahydro-[1,4]diazépino[6,7,1-hi]indol-3-yl)-amide

### [(I); A = 3-quinolyl, R = CH₃]

Le composé est préparé selon le procédé A à partir de l'intermédiaire 2.a : (3R,S) 3-amino-9-méthyl-1-phényl-6,7-dihydro-[1,4]diazépino[6,7,1-hi]indol-4-one et du chlorure de l'acide quinoline-3 carboxylique. Rdt = 56% - solide blanc -F= 238°C
Analyse conforme pour C₂₈H₂₂N₄O₂, 0,2 H₂O - C.C.M.: S.A4; 0,18.
R.M.N.¹H δ(ppm): 2,45 (s, 3H); 3,1 (m, 1H); 3,45 (m, 1H); 3,95 (m, 1H); 4,65 (m, 1H); 5,71 (d, 1H s. par éch.); 7,25-7,65 (m, 8H); 7,85 (m, 1H); 7,95 (d, 1H); 8,2 (d, 1H éch.); 8,25 (d, 1H); 8,8 (s, 1H); 9,45 (s, 1H)
I.R.: 3450(large), 3200, 3005, 1690, 1660, 1530, 1430, 1375, 1290, 1245, 1160,920, 780,695 cm⁻¹

### Exemple 4 : (3R) Isoquinoline-3-carboxylic acid (9-méthyl-4-oxo-1-phényl-3,4,6,7-tetrahydro-[1,4]diazépino[6,7,1-hi]indol-3-yl)-amide

### [(I); A = 3-isoquinolyl, R = CH₃]

Le composé est préparé selon le procédé B.b par un mode opératoire identique à l'exemple 2.V avec l'intermédiaire 2.b :(3R)3-amino-9-méthyl-1-phényl-6,7-dihydro-[1,4]diazépino[6,7,1-hi]indol-4-one.
Rdt = 93% - solide blanc -F= 130°C - [α]_{D} = +8° (c = 1, CH₂Cl₂)
Analyse conforme pour C₂₇H₂₀N₄O₂, 0,4 H₂O, 0,15 CH₂Cl₂ - C.C.M.: S.A4; 0,17.
R.M.N.¹H δ(ppm): 2,35 (s, 3H); 3,1 (m, 1H); 3,35 (m, 1H); 4,0 (m, 1H); 4,65 (m, 1H); 5,7 (d, 1H s. par éch.); 7,25-7,5 (m, 5H); 7,6 (m, 2H); 7,75 (m, 2H); 8 (d, 1H); 8,1 (d, 1H); 8,65 (s, 1H); 8,8 (s, 1H); 9,9 (d, 1H éch.)
I.R.: 3380, 1660, 1490, 1350, 1235, 1160, 740, 695 cm⁻¹

### Exemple 5 : (3R,S) Isoquinoline-3-carboxylic acid (9-méthoxy-4-oxo-1-phényl-3,4,6,7-tetrahydro-[1,4]diazépino[6,7,1-hi]indol-3-yl)-amide

### [(I); A = 3-isoquinolyl, R = CH₃O]

Le composé est préparé selon le procédé A à partir de l'intermédiaire 3.a : (3R,S) 3-amino-9-méthoxy-1-phényl-6,7-dihydro-[1,4]diazépino[6,7,1-hi]indol-4-one et du chlorure de l'acide quinoline-3 carboxylique. Rdt = 40% - solide blanc -F= 204°C
Analyse conforme pour C₂₈H₂₂N₄O₃; 0,33 H₂O, 0,33 CH₂Cl₂. - C.C.M.: S.B1; 0,25.
R.M.N.¹H δ(ppm): 3,1 (m, 1H); 3,35 (m, 1H); 3,75 (s, 3H); 4,0 (m, 1H); 4,7 (m, 1H); 5,7 (d, 1H s. par éch.); 6,7 (s.large, 1H); 7,1 (s.large, 1H); 7,25-7,85 (m, 7H); 7,95 (d, 1H); 8,2 (d, 1H); 8,25 (d, 1H éch.); 8,75 (m, 1H); 9,75 (m, 1H).
I.R.: 3300(large), 1660, 1520, 1480, 1390, 1290, 1230, 1120, 780, 700 cm⁻¹

### Exemple 6.A : (3R) 3-Chloro-N-(9-méthoxy-4-oxo-1-phényl-3,4,6,7-tétrahydro-[1,4]diazépino[6,7,1-hi]indol-3-yl)-benzamide

### [(I); A = 3-chlorophényl, R = CH₃O]

Préparé selon le procédé C à partir de l'intermédiaire 3.b : (3R) 3-amino-9-méthoxy-1-phényl-6,7-dihydro-[1,4]diazépino[6,7,1-hi]indol-4-one et de l'acide 3-chlorobenzoïque.
Rdt = 75,9% - solide amorphe - F=119°C - [α]_{D} = +29° (c = 1, CH₂Cl₂)
Analyse conforme pour C₂₅H₂₀ClN₃O₃ - C.C.M.: S.A2; 0,59.
R.M.N.1H δ(ppm): 3,10 (m, 1H); 3,35 (m, 1H); 3,70 (s, 3H); 4,00 (m, 1H); 4,65 (m, 1H); 5,60 (d, 1H); 6,70 (s, 1H°; 7,10 (s, 1H); 7,35-7,65 (m, 7H); 7,85 (d, 1H); 8,00 (s, 1H); 8,05 (d, 1H éch.)
I.R.: 3300, 1660, 1570, 1510, 1460, 1370, 1340, 1260, 1230, 1170, 1140, 1040, 760, 740, 700 cm⁻¹

### Exemple 6.B : (3R) 4-Chloro-N-(9-méthoxy-4-oxo-1-phényl-3,4,6,7-tétrahydro-[1,4]diazépino[6,7,1-hi]indol-3-yl)-benzamide

### [(I); A = 4-chlorophényl, R = CH₃O]

Préparé selon le procédé A à partir de l'intermédiaire 3.b et de chlorure de 4-chlorobenzoyle.
Rdt = 83% - solide blanc - F=175°C - [α]_{D} = +3,26° (c = 1, CH₂Cl₂)
Analyse conforme pour C₂₅H₂₀ClN₃O_{3,},0,25H₂O - C.C.M.: S.A3; 0,49.
R.M.N.¹H δ(ppm)= 3,1 (m, 1H); 3,35 (m, 1H); 3,75 (s, 3H); 4,0 (m, 1H); 4,65 (m, 1H); 5,6 (d, 1H s. par éch.); 6,7 (s, 1H); 7,1 (s, 1H);7,35-7,6 (m, 7H);7,9 (d, 2); 8,0 (d, 1H éch.).
I.R.: 3300, 2900, 1650, 1470, 1365, 1340, 1265, 1230, 1140, 1085, 840, 750, 700 cm⁻¹

### Exemple 6.C : (3R) N-(9-méthoxy-4-oxo-1-phényl-3,4,6,7-tétrahydro-[1,4]diazépino[6,7,1-hi]indol-3-yl)-isonicotinamide

### [(I); A = 4-pyridyl, R = CH₃O]

Composé préparé selon le procédé B.a à partir de l'intermédiaire 3.b et de l'ester pentafluorophénylique intermédiaire obtenu avec l'acide isonicotinique.

Rdt = 55% - produit amorphe jaune - F = 220-224°C - [α]_{D} = +2,4° (c = 1, CH₂Cl₂) Analyse conforme pour C₂₄H₂₀N₄O₃, 0,15 CH₂Cl₂ - C.C.M.: S.A3; 0,42.
R.M.N.¹H δ(ppm): 3,15 (m, 1H); 3,35 (m, 1H); 3,75 (s, 3H); 4,00 (m, 1H); 4,65 (m, 1H); 5,60 (d, 1H s. par éch.); 6,70 (s, 1H); 7,10 (s, 1H); 7,25-7,60 (m, 5H); 7,80 (d,
2H); 8,10 (d, 1H éch.); 8,80 (d, 2H)
I.R.: 3350, 1685, 1650, 1525, 1570, 1460, 1230, 695 cm⁻¹

### Exemple 6.D : (3R) Quinoline-3-carboxylic acid (9-méthoxy-4-oxo-1-phényl-3,4,6,7-tetrahydro-[1,4]diazépino[6,7,1-hi]indol-3-yl)-amide

### [(I); A = 3-quinolyl, R = CH₃O]

Le composé est préparé selon le procédé B.b par un mode opératoire identique à l'exemple 2.V avec l'intermédiaire 3.b et l'intermédiaire ester pentafluorophénylique de l'acide quinoline-3-carboxylique.

Rdt = 77% - solide blanc -F= 112°C - [α]_{D} = +4,6° (c = 1, CH₂Cl₂)
Analyse conforme pour C₂₈H₂₂N₄O₃, 0,66 H₂O - C.C.M.: S.B1; 0,30
R.M.N.¹H δ(ppm): 3,1 (m, 1H); 3,35 (m, 1H); 3,75 (s, 3H); 4,0 (m, 1H); 4,7 (m, 1H); 5,7 (d, 1H s. par éch.); 6,7 (s.large, 1H); 7,1 (s.large, 1H); 7,25-7,85 (m, 7H); 7,95 (d, 1H); 8,2 (d, 1H); 8,25 (d, 1H éch.); 8,75 (m, 1H); 9,75 (m, 1H).
I.R.: 3300, 1660, 1520, 1480, 1390, 1290, 1230, 1120, 780, 700 cm⁻¹

### Exemple 6.E : (3R) Ouinoline-6-carboxylic acid (9-méthoxy-4-oxo-1-phényl-3,4,6,7-tetrahydro-[1,4]diazépino[6,7,1-hi]indol-3-yl)-amide.

### [(I); A = 6-quinolyl, R = CH₃O]

Le composé est préparé selon le procédé B.b par un mode opératoire identique à l'exemple 2.V avec l'intermédiaire 3.b et l'intermédiaire ester pentafluorophénylique de l'acide quinoline-6-carboxylique.

Rdt = 80% - solide blanc - F= 206°C - [α]_{D} = +2,95° (c = 1, CH₂Cl₂)
Analyse conforme pour C₂₈H₂₂N₄O₃, H₂O - C.C.M.: S.A10; 0,43.
R.M.N.¹H δ(ppm): 3,15 (m, 1H); 3,4 (m, 1H); 3,75 (s, 3H); 4,0 (m, 1H); 4,7 (m, 1H); 5,65 (d, 1H s. par éch.); 6,7 (s, 1H); 7,1 (s, 1H); 7,35-7,65 (m, 6H); 8,2-8,35 (m, 4H, 1H éch.); 8,5 (s, 1H); 9 (m, 1H).
I.R.: 3400(large), 1650, 1470, 1370, 1345, 1270, 1230, 1190, 1140, 840, 780, 700 cm ⁻¹

### Exemple 6. F : (3R) Isoquinoline-3-carboxylic acid (9-méthoxy-4-oxo-1-phényl-3,4,6,7-tetrahydro-[1,4]diazépino[6,7,1-hi]indol-3-yl)-amide

### [(I); A = 3-isoquinolyl, R = CH₃O]

Le composé est préparé selon le procédé B.b par un mode opératoire identique à l'exemple 2.V avec l'intermédiaire 3.b et l'intermédiaire ester pentafluorophénylique de l'acide isoquinoline-3-carboxylique.
Rdt = 87% - solide blanc - F= 211°C - [α]_{D} = +0,30° (c = 1, CH₂Cl₂)
Analyse conforme pour C₂₈H₂₂N₄O₃, 0,1 H₂O, 0,1 CH₂Cl₂ - C.C.M.: S.A1; 0,18. R.M.N.¹H δ(ppm): 3,1 (m, 1H); 3,35 (m, 1H); 3,75 (s, 3H); 4,0 (m, 1H); 4,7 (m, 1H); 5,7 (d, 1H s. par éch.); 6,7 (s.large); 7,1 (s.large, 1H); 7,2-7,8 (m, 7H); 8,0 (m, 1H); 8,1 (m, 1H); 8,65 (s, 1H); 9,3 (s, 1H); 9,9 (d, 1H éch.).
I.R.: 3360, 1665, 1500, 1490, 1470,1345, 1265, 1225, 1145, 700 cm⁻¹

### Exemple 6.G : (3R) 4,7-Diméthyl-pyrazolo[5,1-c][1,2,4]triazine-3-carboxylic acid (9-méthoxy-4-oxo-1-phényl-3,4,6,7-tetrahydro-[1,4]diazépino[6,7,1-hi]indol-3-yl)-amide

### [(I); A = 3-(4,7-Diméthyl-pyrazolo[5,1-c][1,2,4]triazinyl), R = CH₃O]

Le composé est préparé selon le procédé C avec l'intermédiaire 3.b et l'acide 4,7-Diméthyl-pyrazolo[5,1-c][1,2,4]triazine-3-carboxylique.
Rdt = 60,9% - solide amorphe -F= 95°C - [α]_{D} = +19,6° (c = 1, CH₂Cl₂)
Analyse conforme pour C₂₆H₂₃N₇O₃ - C.C.M.: S.A9; 0,82.
R.M.N.¹H δ(ppm) : 2,60 (s, 3H); 3,05 (m, 1H); 3,25 (s, 3H); 3,30 (m, 1H); 3,65 (s, 3H); 3,90 (m, 1H); 4,60 (m, 1H); 5,60 (d, 1H); 6,60 (s, 1H); 7,00 (s, 2H); 7,25-7,45 (m, 3H); 7,55 (m, 2H); 9,80 (d, 1H éch.)
I.R.: 3350, 1670, 1570, 1530, 1490, 1440, 1370, 1350, 1300, 1260, 1230, 1140, 1040, 800, 780, 740, 700 cm⁻¹

### Exemple 7.A : (3R) 2,4-difluoro-N-(4-oxo-1-phénvl-3,4,6,7-tétrahydro-[1,4] diazépino[6,7,1-hi] indol-3-yl)-benzamide

### [(I) ; A = 2,4-difluorophényl]

Composé préparé selon le procédé A à partir de l'intermédiaire 1.b et du chlorure de 2,4-difluorobenzoyle.
Rdt = 88 % - solide blanc - F = 172 °C - [α]_{D} = + 42° (c = 1, CH₂Cl₂)
Analyse conforme pour C₂₄H₁₇N₃O₂F₂ - C.C.M. : S.A3; 0,83.
R.M.N.¹H δ(ppm) : 3,15 (m,1H); 3,35 (m,1H); 4,0 (m,1H), 4,65 (m,1H); 5,55 (d,1H); 6,95 (m, 1H); 7,0 (m,1H); 7,12 (m,1H); 7,25 (m,1H); 7,35 (m,2H); 7,45 (m,2H); 7,53 (m,2H); 8,15 (m,1H); 8,50 (m, 1H éch.).

### Exemple 7.B : (3R) 2,5-difluoro-N-(4-oxo-1-phényl-3,4,6,7-tétrahydro-[1,4] diazépino[6,7,1-hi] indol-3-yl)-benzamide

### [(I) ; A = 2,5-difluorophényl]

Composé préparé selon le procédé A à partir de l'intermédiaire 1.b et du chlorure de 2,5-difluorobenzoyle.
Rdt = 92 % - solide blanc - F = 187 ° C - [α]_{D} = + 42 ° (c = 1, CH₂Cl₂)
Analyse conforme pour C₂₄H₁₇N₃ O₂F₂ - C.C.M. : S.A3; 0,80.
R.M.N.¹H δ(ppm) : 3,14 (m,1H); 3,35 (m,1H); 3,95 (m,1H), 4,65 (m,1H); 5,13 (d,1H); 7,15 (m, 3H); 7,25 (m,1H); 7,35 (m,2H); 7,45 (m,2H); 7,55 (m,2H); 7,80 (m,1H); 8,6 (m,1H éch.).

### Exemple 7.C : (3R) 3,4-difluoro-N-(4-oxo-1-phényl-3,4,6,7-tétrahydro-[1,4] diazépino[6,7,1-hi] indol-3-yl)-benzamide

### [(I) ; A = 3,4-difluorophényl]

Composé préparé selon le procédé A à partir de l'intermédiaire 1.b et du chlorure de 3,4-difluorobenzoyle.
Rdt = 77 % - solide blanc - F = 236 °C - [α]_{D} = + 46 ° (c = 1, CH₂Cl₂) Analyse conforme pour C₂₄H₁₇N₃ O₂F₂ - C.C.M. : S.A3 ; 0,70.
R.M.N.¹H δ(ppm) : 3,15 (m,1H); 3,35 (m,1H); 4,0 (q,1H), 4,65 (m,1H); 5,58 (d,1H); 7,12 (m, 1H); 7,25 (m,2H); 7,35 (m,2H); 7,45 (m,2H); 7,55 (m,2H); 7,72 (m,1H); 7,82 (m,1H); 7,95 (d, 1H éch.).
I.R. : 3300, 1680, 1630, 1600,1540, 1500, 1280, 1250, 780, 690 cm⁻¹

### Exemple 7.D : (3R) 3,5-difluoro-N-(4-oxo-1-phényl-3,4,6,7-tétrahydro-[1,4] diazépino[6,7,1-hi]indol-3-yl)-benzamide

### [(I) ; A = 3,5-difluorophényl]

Composé préparé selon le procédé A à partir de l'intermédiaire 1.b et du chlorure de 3,5-difluorobenzoyle.
Rdt = 90 % - solide jaune pâle - F = 279 °C - [α]_{D} = + 44 ° (c = 1, CH₂Cl₂) Analyse conforme pour C₂₄H₁₇N₃O₂F₂ - C.C.M. : S.A3 ; 0,70.
R.M.N.¹H δ(ppm) : 3,15 (m,1H); 3,35 (m,1H); 4,0 (m,1H), 4,65 (m,1H); 5,60 (d,1H); 6,95 (m, 1H); 7,10 (m,1H) 7,48 (m,2H); 7,45 (m,6H); 8,0 (d,1H éch.).
I.R. : 3200, 1680, 1640, 1590,1540, 1440, 1380, 1300, 1120, 990, 840, 690 cm⁻¹

### Exemple 7.E : (3R) 2,4-dichloro-N-(4-oxo-1-phényl-3,4,6,7-tétrahydro-[1,4] diazépino[6,7,1-hi] indol-3-yl)-benzamide

### [(I) ; A = 2,4-dichlorophényl]

Composé préparé selon le procédé A à partir de l'intermédiaire 1.b et du chlorure de 2,4-dichlorobenzoyle.
Rdt = 77 % - solide jaunâtre - F = 186 °C - [α]_{D} = + 78 ° (c = 1, CH₂Cl₂)
Analyse conforme pour C₂₄H₁₇N₃ O₂Cl₂ - C.C.M. : S.A3; 0,64.
R.M.N.¹H δ(ppm) : 3,15 (m,1H); 3,38 (m,1H); 4,0 (m,1H) 4,20 (m,1H); 5,63 (d,1H); 7,15 (t, 1H); 7,25 (m,1H); 7,36 (m,3H); 7,45 (m,3H); 7,55 (m,2H); 7,80 (d, 1H); 8,10 (d, 1H éch.).

### Exemple 7. F : (3R) 3,5-dichloro-N-(4-oxo-1-phényl-3,4,6,7-tétrahydro-[1,4] diazépino[6,7,1-hi] indol-3-yl)-benzamide

### [(I) ; A = 3,5-dichlorophényl]

Composé préparé selon le procédé A à partir de l'intermédiaire 1.b et du chlorure de 3,5-dichlorobenzoyle.
Rdt = 65 % - solide jaune -F = 211 °C - [α]_{D} = + 46 ° (c = 1, CH₂Cl₂)
Analyse conforme pour C₂₄H₁₇N₃ O₂Cl₂ - C.C.M. : S.A3 ; 0,85.
R.M.N.¹H δ(ppm) : 3,12 (m,1H); 3,35 (m,1H); 3,95 (m,1H), 4,65 (m,1H); 5,56 (d,1H); 7,10 (m, 1H); 7,20 (m,1H); 7,35 (m,1H); 7,45 (m,6H); 7,8 (s, 2H); 8,12 (d, 1H éch.). I.R. : 3300, 1680, 1650, 1560,1530, 1440, 1400, 1280, 1240, 1120, 800, 700 cm⁻¹

### Exemple 7.G : (3R) 3,5-bis(trifluorométhyl)-N-(4-oxo-1-phényl-3,4,6,7-tétrahydro-[1,4] diazépino[6,7,1-hi] indol-3-yl)-benzamide

### [(I) ; A = 3,5-bis(trifluorométhyl)-phényl]

Composé préparé selon le procédé A à partir de l'intermédiaire 1.b et du chlorure de 3,5-bis(trifluorométhyl)-benzoyle. Rdt = 92 % - solide jaunâtre - F = 208 °C - [α]_{D} = + 46 ° (c = 1, CH₂Cl₂)
Analyse conforme pour C₂₆H₁₇N₃ O₂F₆ - C.C.M. : S.A3 ; 0,88.
R.M.N.¹H δ(ppm) : 3,5 (m,1H); 3,35 (m,1H); 3,98 (q,1H), 4,65 (m,1H); 5,60 (d,1H); 7,12 (m, 1H); 7,25 (m,2H); 7,35 (m,2H); 7,45 (m,2H); 7,52 (m, 2H); 7,22 (m, 1H); 7,82 (m,1H); 7,95 (d,1H éch.).
I.R. : 3250, 1650, 1520, 1440, 1380, 1280, 1170, 1130, 910, 700 cm⁻¹

### Exemple 7.H : (3R) 3,4-diméthoxy-N-(4-oxo-1-phényl-3,4,6,7-tétrahydro[1,4] diazépino[6,7,1-hi] indol-3-yl)-benzamide

### [(I) ; A = 3,4-diméthoxyphényl]

Composé préparé selon le procédé A à partir de l'intermédiaire 1.b et du chlorure de 3,4-diméthoxybenzoyle.
Rdt = 91 % - solide blanc - F = 144 °C - [α]_{D} = + 54 ° (c = 1, CH₂Cl₂)
Analyse conforme pour C₂₆H₂₃N₃ O₄ - C.C.M. : S.A3 ; 0,33
R.M.N.¹H δ(ppm) : 3,15 (m,1H); 3,35 (m,1H); 3,95 (d,6H), 3,95 (m,1H); 4,65 (t,1H); 5,65 (d, 1H); 6,90 (d,1H); 7,10 (t,1H); 7,25 (t,1H); 7,35 (t, 2H); 7,45 (m, 2H); 7,55 (m,4H); 7,95 (d,1H éch.).
I.R. : 3300, 1680, 1650, 1600, 1490, 1440, 1260, 1220, 1020, 760, 700 cm⁻¹

### Exemple 7.I : (3R) 3-chloro-4-méthoxy-N-(4-oxo-1-phényl-3,4,6,7-tétrahydro-[1,4] diazépino[6,7,1-hi] indol-3-yl)-benzamide

### [(I) ; A = 3-chloro-4-méthoxy-phényl]

Composé préparé selon le procédé C à partir de l'intermédiaire 1.b et de l'acide 3-chloro-4-méthoxy-benzoïque.
Rdt = 82 % - solide rose - F = 158 °C - [α]_{D} = + 50 ° (c = 1, CH₂Cl₂)
Analyse conforme pour C₂₅H₂₀N₃ O₃Cl - C.C.M. : S.A3 ; 0,60.
R.M.N.¹H δ(ppm) : 3,12 (m,1H); 3,35 (m,1H); 3,95 (s,3H), 4,0 (m,1H); 5,10 (d,1H); 6,95 (d, 1H); 7,10 (t,1H); 7,25 (m,1H); 7,35 (m,2H); 7,43 (m, 2H); 7,50 (m, 2H); 7,85 (m,1H); 7,95 (m,1H éch.); 8,15 (d,1H).
I.R. : 3300, 1650, 1600, 1480, 1440, 1390, 1260, 1060, 760, 700 cm⁻¹

### Exemple 7.J : (3R) 4-amino-3,5-dichloro-N-(4-oxo-1-phényl-3,4,6,7-tétrahydro-[1,4] diazépino[6,7,1-hi] indol-3-yl)-benzamide

### [(I) ; A = 4-amino-3,5-dichlorophényl]

Composé préparé selon le procédé C à partir de l'intermédiaire 1.b et de l'acide 4-amino-3,5-dichlorobenzoïque.
Rdt = 90 % - solide saumon - F = 168 °C -[α]_{D} = + 54 ° (c = 1, CH₂Cl₂)
Analyse conforme pour C₂₄H₁₈N₄O₂Cl₂ - C.C.M. : S.A3 ; 0,62
R.M.N.¹H δ(ppm) : 7,9 (s,1H); 7,3 (m,8H); 5,6 (d,1H), 4,9 (s,2H); 4,7 (t,1H); 4 (q, 1H); 3,4 (m,1H); 3,1 (m,1H).
I.R. : 3300, 1600, 1520, 1470, 1390, 1350, 1280, 1120, 780, 700 cm⁻¹

### Exemple 7.K : (3R) 2-acétamido-N-(4-oxo-1-phényl-3,4,6,7-tétrahydro-[1,4] diazépino[6,7,1-hi] indol-3-yl)-benzamide

### [(I) ; A = 2-acétamido-phényl]

Composé préparé selon le procédé C à partir de l'intermédiaire 1.b et de l'acide N-acétylanthranilique.
Rdt = 27 % - solide blanc - F = 210 °C - [α]_{D} = + 46 ° (c = 1, CH₂Cl₂)
Analyse conforme pour C₂₆H₂₂N₄ O₃ - C.C.M. : S.A9 ; 0,26
R.M.N.¹H δ(ppm) : 1,75 (s large,éch.); 2,85 (s,3H); 3,15 (m,1H); 3,4 (m,1H); 4,0 (m,1H); 4,22 (m, 1H); 7,12 (m,1H); 7,20 (s,1H) ; 7,25 (m,1H); 7,45 (5H); 7,55 (d,2H); 7,75 (m,2H); 8,2 (d,1H).

### Exemple 7.L: (3R)2-acétoxy-N-(4-oxo-1-phényl-3,4,6,7-tétrahydro-[1,4]diazépino[6,7,1-hi]indol-3-yl)-benzamide

### [(I) ; A = 2-acétoxy-phényl]

Composé préparé selon le procédé C à partir de l'intermédiaire 1.b et de l'acide acétylsalicylique.
Rdt = 14 % - F = 147 °C - [α]_{D} = +57 ° (c = 1, CH₂Cl₂)
Analyse conforme pour C₂₆H₂₁ O₄N₃ - C.C.M. : S.A3 ; 0,50
R.M.N.¹H δ(ppm) : 2,5 (s,3H); 3,12 (m,1H); 3,36 (m,1H), 3,95 (q,1H); 4,5 (t,1H); 5,63 (d,1H); 7,1 (t,1H); 7,7 (d,1H); 7,25 (m,1H); 7,35 (m,3H); 7,45 (m, 2H); 7,62 (m, 3H); 8,18 (d,1H); 8,48 (d,1H éch.).
I.R. : 3400, 1760, 1660, 1600, 1500, 1180, 1090, 1910, 730, 700 cm⁻¹

### Exemple 7.M : (3R) 2-hydroxy-N-(4-oxo-1-phényl-3,4,6,7-tétrahydro-[1,4] diazépino[6,7,1-hi]indol-3-yl)-benzamide sel de potassium

### [(I) ; A = 2-phénate de potassium]

La fonction ester du produit de l'exemple 7.L est saponifiée par la potasse dans le méthanol au reflux pendant 2 h, puis le solvant est évaporé.
Rdt = 89 % - solide jaune - F = 243 ° C - [α]_{D} = + 4 ° (c = 1, CH₂Cl₂)
Analyse conforme pour C₂₄H₁₈O₃N₃K, 2,5H₂O - C.C.M. : S.A3 ; 0,67
R.M.N.¹H δ(ppm) : 3,16 (m,1H) 3,35 (m,1H) 3,5 (H₂O); 3,92 (q,1H); 4,98 (t,1H); 5,1 (s,1H) 6,3 (t,1H); 6,75 (d,1H) 7,02 (m,1H); 7,2 (m, 2H); 12,7 (1H large éch.); 7,48 (m,5H); 7,6 (m, 1H); 7,7 (m, 1H).

### Exemple 7.N : (3R) 5,6-dichloro-N-(4-oxo-1-phényl-3,4,6,7-tétrahydro-[1,4] diazépino[6,7,1-hi] indol-3-yl)-nicotinamide

### [(I) ; A = 3-(5,6-dichloro-pyridyl)]

Composé préparé selon le procédé C à partir de l'intermédiaire 1.b et de l'acide 5,6-dichloro-nicotinique.
Rdt = 49 % - solide blanc - F = 138-140 °C - [α]_{D} = +43,7 ° (c = 1, CH₂Cl₂)
Analyse conforme pour C₂₃H₁₆ Cl₂N₄O₂ - C.C.M. : S.A3 ; 0,30
R.M.N.¹H δ(ppm) : 3,1-3,25 (m,1H); 3,3-3,5 m,1H); 3,95-4,1 (q,1H); 4,6-4,75 (t,1H); 5,5-5,6 (d,1H); 7,1-7,6 (m,8H); 8,1-8,25 (d,1H); 8,25-8,35 (s,1H); 8,75-8,9 (s,1H).
I.R. : 3250, 1650, 1520, 1360, 1290, 1240, 1150, 1040, 760, 690 cm⁻¹

### Exemple 7.O : (3R) 3,5-dichloro-N-(4-oxo-1-phényl-3,4,6,7-tétrahydro-[1,4] diazépino[6,7,1-hi] indol-3-yl)-isonicotinamide

### [(I) ; A = 3,5-dichloro-4-pyridyl]

Composé préparé selon le procédé C à partir de l'intermédiaire 1.b et de l'acide 3,5-dichloro-isonicotinique
Rdt = 18 % - solide blanc - F = 182 °C - [α]_{D} = + 136,5 ° (c = 1, CH₂Cl₂)
Analyse conforme pour C₂₃H₁₆ Cl₂N₄O₂ - C.C.M. : S.A10 ; 0,45.
R.M.N.¹H δ(ppm) : 3,1-3,25 (m,1H); 3,35-3,5 (m,1H); 3,9-4,1 (m,1H); 4,6-4,75 (m,1H); 5,6-5,7 (d,1H); 7,1-7,6 (m,8H); 7,7-7,85 (d,1H); 8,5-8,6 (d,2H)
I.R. : 3300, 1670, 1600, 1520, 1390, 1280, 1200, 880, 820,700 cm⁻¹

### Exemple7. P: (3R)3-t-butyloxycarbonylamino-N-(4-oxo-1-phényl-3,4,6,7-tétrahydro-[1,4] diazépino[6,7,1-hi] indol-3-yl)-isonicotinamide

### [(I) ; A = 3-t-butyloxycarbonylamino-4-pyridyl]

Composé préparé dans le diméthylformamide selon le procédé C décrit à l'exemple 1.B, c'est-à-dire en présence de PyBrop et de triéthylamine, à partir de l'intermédiaire 1.b et du dérivé N-Boc de l'acide 3-amino-isonicotinique.
Rdt = 67 % - solide jaune - C.C.M. : S.A10 ; 0,40.
R.M.N.¹H δ(ppm) : 1,45 (s,9H); 3,15 (m,1H); 3,4 (m,1H); 3,95 (q,1H); 4,5 (t,1H); 5,45 (d,1H); 7,5 (m,9H); 8,4 (d,1H), 9,35 (s,1H); 9,9 (s,1H); 10,2(d,1H).

### Exemple 7.Q : (3R) 3-amino-N-(4-oxo-1-phényl-3,4,6,7-tétrahydro-[1,4] diazépino[6,7,1-hi] indol-3-yl)-isonicotinamide

### [(I) ; A = 3-amino-4-pyridyl]

Le produit de condensation obtenu à l'exemple 7.P précédent est N-déprotégé par l'acide trifluoroacétique dans le chlorure de méthylène. Après 30 min d'agitation à température ambiante, le solvant est éliminé, le résidu repris dans l'acétate d'éthyle, et extrait par une solution saturée de NaHCO₃. Après séchage, le solvant est évaporé et le résidu purifié par chromatographie sur colonne de silice en éluant par le mélange CH₂ Cl₂/MeOH 98/2 (v/v).
Rdt = 68 % - solide beige - F = 175 °C - C.C.M. : S.B ; 0,15
R.M.N.¹H δ(ppm) : 3,15 (m,1H) 3,4 (m,1H); 3,95 (q,1H) 4,5 (t,1H) 5,5 (d,1H 5,75 (s,1H); 6,45 (s,1H); 7,5 (m,9H); 8,2 (s,1H); 9,7 (d,1H)
I.R. : 3300, 2900, 1680, 1640, 1600, 1580, 1500, 1230,1040,700 cm⁻¹

### Exemple 7.R : (3R) 3-acétamido-N-(4-oxo-1-phényl-3,4,6,7-tétrahydro-[1,4] diazépino[6,7,1-hi] indol-3-yl)-isonicotinamide

### [(I) ; A = 3-acétamido-4-pyridyl]

0,2g (0,5 mmol) de produit de l'exemple 7.Q sont dissous dans 2 ml de pyridine ; on ajoute 1 ml d'anhydride acétique et on agite durant 12 h à la température ambiante avant d'ajouter 10 ml d'eau. Après 4 h d'agitation à la température ambiante, on extrait à l'acétate d'éthyle ; la phase organique est lavée par une solution saturée de NaHCO₃ puis séchée. Après évaporation, le résidu est purifié par chromatographie sur colonne de silice en éluant par un mélange de polarité croissante d'acétone dans le chlorure de méthylène. Rdt = 45 % - solide blanc - F = 190 °C - C.C.M. : S.B ; 0,17.
R.M.N.¹H δ(ppm) : 2,2 (s,3H); 3,2 (m,1H); 3,4 (m,1H); 4 (q,1H); 4,7 (t,1H); 5,6 (d,1H); 7,3 (m,9H); 8,3 (d,1H); 8,45 (d,1H); 9,9 (s,1H); 10,5 (s, 1H)
I.R. : 3300, 1680, 1650, 1600, 1560, 1500, 1410, 1280, 1240, 700 cm⁻¹

### Exemple 7.S : (3R) 3-cyclopropylcarbonylamino-N-(4-oxo-1-phényl-3,4,6,7-tétrahydro-[1,4] diazépino[6,7,1-hi] indol-3-yl)-isonicotinamide

### [(I) ; A = 3-cyclopropylcarbonylamino-4-pyridyl]

0,15 g (0,38 mmol) de produit de l'exemple 7.Q sont dissous dans 2 ml de chlorure de méthylène ; on ajoute un équivalent de triéthylamine. On refroidit et on ajoute, à une température inférieure à 5 °C, 40 mg (0,38 mmol) de chlorure de cyclopropanecarbonyle. On agite durant 16 h à la température ambiante avant d'ajouter 20 ml de chlorure de méthylène. Après lavage à la soude 1N, séchage et évaporation du solvant, le résidu est purifié par chromatographie sur colonne de silice en éluant par le mélange S.A8
Rdt = 20 % - C.C.M. : S.A10 ; 0,17
R.M.N.¹H δ(ppm) : 0,85 (m,2H); 1,1 (m,2H); 1,6 (m,1H); 3,15 (m,1H); 3,4 (m,1H); 4 (q,1H); 4,7 (t,1H); 5,6 (d,1H); 7,3 (m,9H); 8,3 (d,1H) 8,4 (d, 1H); 9,9 (s, 1H); 10,35 (s,1H).

### Exemple 7.T : (3R) pyrazine-2-carboxylic acid (4-oxo-1phényl-3,4,6,7-tétrahydro-[1,4] diazépino[6,7,1-hi] indol-3yl)-amide

### [(I) ; A = 2-pyrazinyl]

Composé préparé selon le procédé C à partir de l'intermédiaire 1.b et de l'acide 2-pyrazine carboxylique.
Rdt = 72 % - solide orange clair - F = 213-214 °C - [α]_{D} = + 56 ° (c = 1, CH₂Cl₂)- Analyse conforme pour C₂₂H₁₇N₅O₂ - C.C.M. : S.A10 ; 0,75
R.M.N.¹H δ(ppm) : 3,1-3,25 (m,1H) 3,3-3,5 (m,1H); 3,9-4,1 (m,1H); 4,6-4,75 (m,1H); 5,55-5,7 (d,1H); 7,7 (m,8H); 8,6-8,7 (d,1H); 8,7-8,85 (d,1H); 9,35-9,6 (m,2H).
I.R. : 3370, 1670, 1600, 1510, 1450, 1390, 1020, 800, 690 cm⁻¹

### Exemple 7.U : (3R) thiophène-2-carboxylic acid (4-oxo-1phényl-3,4,6,7-tétrahydro-[1,4] diazépino[6,7,1-hi] indol-3yl)-amide

### [(I) ; A = 2-thiényl]

Composé préparé selon le procédé C à partir de l'intermédiaire 1.b et de l'acide 2-thiophène carboxylique.
Rdt = 57 % - solide blanc - F = 217-218 °C - [α]_{D} = +56 ° (c = 1, CH₂Cl₂) Analyse conforme pour C₂₂H₁₇N₃O₂S - C.C.M. : S.A8 ; 0,50
R.M.N.¹H δ(ppm) : 3,1-3,25 (m,1H); 3,25-3,5 (m,1H); 3,9-4,1 (m,1H); 4,6-4,75 (m,1H); 5,5-5,7 (d,1H); 7,0-7,6 (m,10H); 7,65-7,75 (d,1H); 7,75-7,9 (d,1H).
I.R. : 3250, 1690, 1630, 1540, 1440, 1380, 1270, 1160, 710, 520 cm⁻¹

### Exemple 8.A : (3R) 4-chloro-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-quinolyl)]

Composé préparé selon le procédé C décrit à l'exemple 1.B à partir de l'intermédiaire 1.b et de l'acide 4-chloro-quinoline-3-carboxylique.
Rdt = 27 % - solide orange - F = 192 °C
Analyse conforme pour C₂₇H₁₉ClN₄O₂ - C.C.M. : S.B ; 0,48
R.M.N.¹H δ(ppm) : 3,1 (m,1H); 3,35 (m,1H); 3,9 (q,1H); 4,6 (t,1H); 5,6 (d,1H); 7,3 (m,8H); 7,6 (t,1H); 7,7 (t,1H); 8,1 (d,1H); 8,25 (d,1H); 8,35 (d,1H); 9,15 (s,1H).
I.R. : 3200, 1650, 1600, 1500, 1440, 1340, 1240, 840, 760,700 cm⁻¹

### Exemple 8.B : (3R)4-chloro-6-fluoro-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-6-fluoro-quinolyl)]

Composé préparé selon le procédé C décrit à l'exemple 1.B à partir de l'intermédiaire 1.b et de l'acide 4-chloro-6-fluoro-quinoline-3-carboxylique.
Rdt = 29 % - solide orange - F = 191 °C - [α]_{D} = + 93 ° (c = 1, CH₂Cl₂)
Analyse conforme pour C₂₇H₁₈ ClFN₄O₂ - C.C.M. : S.B ; 0,60
R.M.N.¹H δ(ppm) : 3,1 (m,1H) 3,4 (m,1H); 3,9 (q,1H); 4,6 (t,1H) 5,6 (d,1H) 7,3 (m,9H); 7,9 (d, 1H); 8,1 (d,1H); 8,2 (d,1H) 9,1 (s, 1H).
I.R. : 3250, 1660, 1600, 1580, 1490, 1440, 1340,1295, 830,700 cm⁻¹

### Exemple 8.C : (3R) 4,6-dichloro-quinoline-3-carboxylic acid (4-oxo-1-phényl-3,4,6,7-tétrahydro-[1,4] diazépino[6,7,1-hi] indol-3-yl)-amide

### [(I) ; A = 3-(4,6-dichloro-quinolyl)]

Composé préparé selon le procédé C décrit à l'exemple 1.B à partir de l'intermédiaire 1.b et de l'acide 4,6-dichoro-quinoline-3-carboxylique.
Rdt = 31 % - solide jaune - F = 200 °C - [α]_{D} = + 89 ° (c = 1, CH₂Cl₂)
Analyse conforme pour C₂₇H₁₈Cl₂FN₄O₂ - C.C.M. : S.B ; 0,55
R.M.N.¹H δ(ppm) : 3,2 (m,1H); 3,4 (m,1H) 4 (q,1H); 4,7 (t,1H); 5,7 (d,1H); 7,3 (m,8H); 7,7 (d,1H); 8,1 (d,1H 8,35 (m,2H); 9,2 (s,1H).
I.R. : 3500, 1660, 1600, 1580, 1480, 1440, 1330, 1240,820, 700 cm⁻¹

### Exemple 8.D : (3R) 4,8-dichloro-quinoline-3-carboxylic acid (4-oxo-1-phényl-3,4,6,7-tétrahydro-[1,4] diazépino[6,7,1-hi] indol-3-yl)-amide

### [(I) ; A = 3-(4,8-dichloro-quinolyl)]

Composé préparé selon le procédé C décrit à l'exemple 1.B à partir de l'intermédiaire 1.b et de l'acide 4,8-dichoro-quinoline-3-carboxylique.
Rdt = 33 % - solide jaune - F = 225 °C - [α]_{D} = + 81 ° (c = 1, CH₂Cl₂)
Analyse conforme pour C₂₇H₁₈ Cl₂N₄O₂ - C.C.M. : S.B ; 0,90
R.M.N.¹H δ(ppm) : 3,1 (m,1H); 3,4 (m,1H); 3,95 (q,1H); 4,2 (t,1H) 5,7 (d,1H) 7,4 (m,9H); 7,95 (d, 1H); 8,3 (d,1H); 8,4 (d,1H); 9,3 (s, 1H)
I.R. : 3200, 1660, 1600, 1510, 1465, 1440, 1390, 1340, 750, 700 cm⁻¹

### Exemple 8.E : (3R) 4-chloro-6-bromo-quinoline-3-carboxylic acid (4-oxo-1-phényl 3,4,6,7-tétrahvdro-[1,4] diazépino[6,7,1-hi] indol-3-yl)-amide

### [(I) ; A = 3-(4-chloro-6-bromo-quinolyl)]

Composé préparé selon le procédé C décrit à l'exemple 1.B à partir de l'intermédiaire 1.b et de l'acide 4-chloro-6-bromo-quinoline-3-carboxylique.
Rdt = 33 % - solide jaune - F = 191 °C - [α]_{D} = + 86 ° (c = 1, CH₂Cl₂)
Analyse conforme pour C₂₇H₁₈ BrClN₄O₂ ,0,5H₂O - C.C.M. : S.B ; 0,70
R.M.N.¹H δ(ppm) : 3,15 (m,1H); 3,45 (m,1H); 4 (q,1H); 4,7 (t,1H); 5,7 (d,1H); 7,3 (m,8H); 7,9 (d, 1H); 8 (d,1H); 8,4 (d,1H); 8,55 (s, 1H); 9,2 (s,1H).
I.R. : 3250, 1660, 1600, 1580, 1500, 1470,1330, 1220, 820, 700 cm⁻¹

### Exemple 8.F : (3R) 4-chloro-6-méthyl-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-6-méthyl-quinolyl)]

Composé préparé selon le procédé C en présence de TOTU et de di-isopropyl-éthylamine à partir de l'intermédiaire 1.b et de l'acide 4-chloro-6-méthyl-quinoline-3-carboxylique.
Rdt = 29 % - solide jaune - F = 196 °C - [α]_{D} = + 89 ° (c = 1, CH₂Cl₂)
Analyse conforme pour C₂₈H₂₁ ClN₄O₂ ,0,33H₂O - C.C.M. : S.B ; 0,55
R.M.N.¹H δ(ppm) : 2,6 (s.1H); 3,2 (m,1H); 3,4 (m,1H); 4 (q,1H); 4,7 (t,1H); 5,7 (d,1H); 7,4 (m, 9H); 8,05 (d,1H); 8,1 (s,1H); 8,4 (d,1H); 9,1 (s,1H).
I.R. : 3250, 1650, 1600, 1580, 1490, 1440, 1340, 1240, 820, 700 cm⁻¹

### Exemple 8. G : (3R) 4-chloro-8-méthyl-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-8-méthyl-quinolyl)]

Composé préparé selon le procédé C décrit à l'exemple 1.B à partir de l'intermédiaire 1.b et de l'acide 4-chloro-8-méthyl-quinoline-3-carboxylique.
Rdt = 30 % - solide jaune - F = 240 °C (déc.) - [α]_{D} = + 82 ° (c = 1, CH₂Cl₂)
Analyse conforme pour C₂₈H₂₁ ClN₄O₂ - C.C.M. : S.B ; 0,76
R.M.N.¹H δ(ppm) : 3,1 (m,1H) 3,4 (m,1H); 3,9 (q,1H) 4,6 (t,1H); 5,6 (d,1H) 7,3 (m,10H); 8,15 (d,1H); 8,3 (d,1H); 9,1 (s,1H).
I.R. : 3200, 1660, 1600, 1520, 1440, 1350, 1240, 830, 760, 700 cm⁻¹

### Exemple 8.H : (3R) 4-chloro-6-méthoxy-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-6-méthoxy-quinolyl)]

Composé préparé selon le procédé C décrit à l'exemple 1.B à partir de l'intermédiaire 1.b et de l'acide 4-chloro-6-méthoxy-quinoline-3-carboxylique.
Rdt = 35 % - solide beige - F = 225 °C - [α]_{D} = + 94 ° (c = 1, CH₂Cl₂) Analyse conforme pour C₂₈H₂₁ ClN₄O₃ - C.C.M. : S.B ; 0,55
R.M.N.¹H δ(ppm) : 3,1 (m,1H); 3,4 (m,1H); 4 (m,4H); 4,7 (t,1H); 5,7 (d,1H); 7,3 (m,10H); 8,1 (d,1H); 8,3 (d,1H); 9,05 (s,1H).
I.R. : 3250, 1640, 1580, 1530, 1490, 1440, 1400, 1230, 820, 700 cm⁻¹

### Exemple 8.I : (3R) 4-chloro-8-méthoxy-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-8-méthoxy-quinolyl)]

Composé préparé selon le procédé C décrit à l'exemple 1.B à partir de l'intermédiaire 1.b et de l'acide 4-chloro-8-méthoxy-quinoline-3-carboxylique.
Rdt = 30 % - solide jaune - F = 265 °C - [α]_{D} = + 91 ° (c = 1, CH₂Cl₂)
Analyse conforme pour C₂₈H₂₁ ClN₄O₃ - C.C.M. : S.B ; 0,20
R.M.N.¹H δ(ppm) : 3,05 (m,1H); 3,3 (m,1H); 3,95 (q,1H); 4,05 (s,3H); 4,6 (t,1H); 5,6 (d,1H) 7,3 (m,10H); 7,8 (d,1H); 8,2 (d,1H); 9,1 (s,1H)
I.R. : 3200, 1655, 1600, 1520, 1360, 1270, 1180, 800, 700 cm⁻¹

### Exemple 8.J : (3R) 4-chloro-5,7-diméthyl-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-5,7-diméthyl-quinolyl)]

Composé préparé selon le procédé C décrit à l'exemple 1.B à partir de l'intermédiaire 1.b et de l'acide 4-chloro-5,7-diméthyl-quinoline-3-carboxylique.
Rdt = 26 % - solide jaune - F = 236 °C - [α]_{D} = + 118 ° (c = 1, CH₂Cl₂) Analyse conforme pour C₂₉H₂₃ ClN₄O₂ - C.C.M. : S.B ; 0,55
R.M.N.¹H δ(ppm) : 2,4 (s,3H); 2,9 (s,3H); 3,05 (m,1H); 3,3 (m,1H); 3,9 (q,1H); 4,6 (t,1H); 5,6 (d,1H); 7,3 (m,9H); 7,7 (s,1H); 8 (d,1H); 8,4 (s, 1H)
I.R. : 3250, 1680, 1660, 1600, 1580, 1520, 1440, 1220, 700 cm⁻¹

### Exemple 8.K : (3R) 4-chloro-5,8-diméthyl-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-5,8-diméthyl-quinolyl)]

Composé préparé selon le procédé C décrit à l'exemple 1.B à partir de l'intermédiaire 1.b et de l'acide 4-chloro-5,8-diméthyl-quinoline-3-carboxylique.
Rdt = 30 % - solide beige - F = 240 °C (déc.) - [α]_{D =} + 76 ° (c = 1, CH₂Cl₂) Analyse conforme pour C₂₉H₂₃ ClN₄O₂ - C.C.M. : S.B ; 0,80
R.M.N.¹H δ(ppm) : 3,05 (m,1H); 3,3 (m,1H); 3,8 (q, 1H); 4,6 (t,1H); 5,6 (d,1H); 7,3 (m,10H); 8,1 (d,1H); 8,9 (s, 1H)
I.R. : 3250, 1660, 1600, 1520, 1440, 1390, 1220, 830, 730, 700 cm⁻¹

### Exemple 8.L : (3R) 4-chloro-6,8-diméthyl-quinoline-3-carboxylic acid (4-oxo-1-phényl-3,4,6,7-tétrahydro-[1,4] diazépino[6,7,1-hi] indo]-3-yl)-amide

### [(I) ; A = 3-(4-chloro-6,8-diméthyl-quinolyl)]

Composé préparé selon le procédé C décrit à l'exemple 1.B à partir de l'intermédiaire 1.b et de l'acide 4-chloro-6,8-diméthyl-quinoline-3-carboxylique.
Rdt = 25 % - solide blanc - F = 280 °C - [α]_{D} = + 87 ° (c = 1, CH₂Cl₂)
Analyse conforme pour C₂₉H₂₃ ClN₄O₂ ,0,33H₂O - C.C.M. : S.B ; 0,85
R.M.N.¹H δ(ppm) : 2,6 (s,3H); 2,8 (s,3H); 3,1 (m,1H); 3,4 (m,1H); 3,9 (q,1H) 4,65 (t,1H); 5,7 (d,1H) 7,3 (m,9H); 7,95 (s, 1H); 8,4 (d, 1H); 9,1 (s,1H)
I.R. : 3250, 1660, 1600, 1520, 1490, 1440, 1360, 1220, 840, 700 cm⁻¹

### Exemple 8. M : (3R) 4-chloro-7,8-diméthyl-quinoline-3-carboxylic acid (4-oxo-1-phényl-3,4,6,7-tétrahydro-[1,4] diazépino[6,7,1-hi] indol-3-yl)-amide

### [(I) ; A = 3-(4-chloro-7,8-diméthyl-quinolyl)]

Composé préparé selon le procédé C décrit à l'exemple 1.B à partir de l'intermédiaire 1.b et de l'acide 4-chloro-7,8-diméthyl-quinoline-3-carboxylique.
Rdt = 35 % - solide jaune - F = 203 °C - [α]_{D} = + 88 ° (c = 1, CH₂Cl₂)
Analyse conforme pour C₂₉H₂₃ ClN₄O₂ ,0,5H₂O - C.C.M. : S.B ; 0,90
R.M.N.¹H δ(ppm) : 3,1 (m,1H); 3,3 (m,1H) 3,9 (q,1H); 4,6 (t,1H); 5,6 (d,1H); 7,3 (m,9H); 8 (d,1H); 8,3 (d,1H); 9,1 (s,1H)
I.R. : 3250, 1660, 1600, 1515, 1440, 1395, 1360, 1230, 780, 700 cm⁻¹

### Exemple 9.A : (3R) 2-méthoxy-N-(9-méthyl-4-oxo-1-phényl-3,4,6,7-tétrahydro-[1,4] diazépino[6,7,1-hi] indol-3-yl)-benzamide

### [(I) ; A = 2-méthoxyphényl, R = CH₃]

Composé préparé selon le procédé A à partir de l'intermédiaire 2.b et du chlorure de 2-méthoxybenzoyle.
Rdt = 80 % - solide jaune pâle - F = 123-125 °C (déc.) - [α]_{D} = + 34 ° (c = 1, CH₂Cl₂) - Analyse conforme pour C₂₆H₂₃ N₃O₃ - C.C.M. : S.A9; 0,66
R.M.N.¹H δ(ppm) : 2,35 (s,3H); 3,1 (m,1H); 3,35 (m,1H); 4 (m,1H); 4,1 (s,3H); 4,65 (m,1H); 5,7 (d,1H); 7-7,5 (m,10H); 8,25 (d,1H); 9,8 (d,1H éch.).
I.R. : 3350, 1680, 1650, 1600, 1510, 1490, 1290, 1240, 1160, 1020, 750, 700 cm⁻¹

### Exemple 9.B: (3R) N-(9-méthyl-4-oxo-1-phényl-3,4,6,7-tétrahydro-[1,4]diazépino[6,7,1-hi] indol-3-yl)-isonicotinamide

### [(I) ; A = 4-pyridyl, R = CH₃]

Composé préparé selon le procédé A à partir de l'intermédiaire 2.b et du chlorure de l'acide isonicotinique.
Rdt = 88 % - solide blanc - F = 238-240 °C - [α]_{D} = + 54 ° (c = 1, CH₂Cl₂)
Analyse conforme pour C₂₄H₂₀ N₄O₂ - C.C.M. : S.A 10; 0,57
R.M.N.¹H δ(ppm) : 2,35 (s,3H); 3,05 (m,1H); 3,25 (m,1H); 3,9 (m,1H) 4,6 (m,1H); 5 (d,1H); 7 (s,1H); 7,2-7,5 (m,6H); 7,7 (d,2H); 8,1 (d,1H éch.); 8,7 (d,2H) .
I.R. : 3700, 1680, 1650, 1520, 1350, 1280, 1230, 1170, 690, 650 cm⁻¹

### Exemple 9.C : (3R) 4,7-diméthyl-pyrazolo[5,1-c] [1,2,4] triazine-3-carboxylic acid (9-méthyl-4-oxo-1-phényl-3,4,6,7-tétrahydro-[1,4]diazépino[6,7,1-hi]indol-3-yl)-amide

### [(I) ; A = 3-(4,7-diméthyl-pyrazolo[5,1-c] [1,2,4] triazinyl), R = CH₃]

Composé préparé selon le procédé C à partir de l'intermédiaire 2.b et de l'acide 4,7-diméthyl-pyrazolo[5,1-*c*[1,2,4] triazine-3-carboxylique.
Rdt = 72 % - solide jaune - F = 130 °C - [α]_{D} = + 20 ° (c = 1, CH₂Cl₂)
Analyse conforme pour C₂₆H₂₃ N₇O₂ - C.C.M. : S.A4 ; 0,37
R.M.N.¹H δ(ppm) : 2,3 (s,3H); 2,5 (s,3H); 3,2 (s,3H); 5,6(d,1H); 9,8 (d,1H)
I.R. : 3100, 2950, 1665 cm⁻¹

### Partie biologique

### - Activité inhibitrice de phosphodiestérase

La capacité des composés de formule (I) de l'invention à inhiber les phosphodiestérases des nucléotides cycliques est évaluée par la mesure de leur CI₅₀ (concentration nécessaire pour inhiber 50 % de l'activité enzymatique). Dans le cas des PDE _{IV}, cette valeur est comparée à la CI₅₀ du rolipram, inhibiteur spécifique de PDE _{IV}, par le rapport CI₅₀ du rolipram sur CI₅₀ du produit à tester vis-à-vis de la même préparation enzymatique.

Les différents types de phosphodiestérases sont obtenus partiellement purifiés sur colonne de DEAE-cellulose à partir de trachée de cobaye et d'aorte de chien selon une méthode adaptée de W.J. Thompson et al., 1979, Advances in Cyclic Nucleotide Research, Vol. 10 : 69-92, ed. G. Brooker et al. Raven Press, New York, et de P.J. Silver et al., 1988, Eur. J. Pharmacol. 150 : 85-94.

Puis la mesure de l'activité enzymatique des différents types de PDE, et en particulier des PDE _{IV}, est faite selon une méthode également adaptée de W.J. Thompson, *Ibidem.*

Pour la détermination de la CI₅₀, l'activité enzymatique est mesurée en présence de l'inhibiteur dans une gamme de concentrations de 0,1 à 100µM.

Le tableau suivant illustre l'activité inhibitrice de PDE _{IV} comparativement à celle du rolipram sur une préparation d'enzyme obtenue à partir de trachée de cobaye.

**Tableau 1 :**

| Effet inhibiteur de PDE _{IV} comparé au rolipram. | | | | | |
|---|---|---|---|---|---|
| Ex. | CI₅₀ rolipram CI₅₀ exemple | Ex. | CI₅₀ rolipram CI₅₀ exemple | Ex. | CI₅₀ rolipram CI₅₀ exemple |
| 1.B | 3,7 | 2.S | 3,3 | 6.D | 1,9 |
| 2.B | 1,2 | 2.T | 2,0 | 6.E | 1,9 |
| 2.C | 1,2 | 2.Y | 1,4 | 6.F | 1,2 |
| 2.M | 1,3 | 5 | 1,2 | 6.G | 2,2 |
| 2.N | 2,5 | 6.A | 2,7 | 7.J | 2,4 |
| 2.O | 1,9 | 6.B | 1,6 | 9.A | 1,4 |
| 2.P | 1,5 | 6.C | 1,6 | 9.C | 1,0 |

L'examen des résultats du tableau précédent montre que les produits de l'invention testés dans l'essai inhibent généralement l'enzyme PDE _{IV} de trachée de cobaye plus efficacement que le rolipram, et dans nombre de cas sont deux à trois fois plus actifs que le rolipram.

Par ailleurs des essais réalisés sur des PDE de différents types, purifiées à partir de trachée de cobaye ou d'aorte de chien, montrent que les valeurs de CI₅₀ obtenues avec les produits de l'invention vis-à-vis des PDE de type III et de type I et V sont beaucoup plus élevées que celles mesurées pour les PDE de type IV.

Ces résultats sont probants d'une activité inhibitrice puissante et sélective, pour les produits de l'invention, sur les PDE _{IV}.

### - Activité anti-inflammatoire et anti-allergique in vivo

Les effets des produits de l'invention ont été étudiés chez le cobaye dans un modèle d'infiltration d'éosinophiles induite par une stimulation antigénique ou par l'exposition à un aérosol de PAF selon une méthodologie décrite par Lagente V. *et al*., (1994) Br. J. Pharmacol. 112, 83P.

L'administration de produits des Exemples (1 - 30 mg/kg p.o.) diminue significativement le nombre d'éosinophiles dans le liquide de lavage broncho-alvéolaire.

L'administration de produits de l'invention diminue également les réponses inflammatoires induites par l'instillation intratrachéale d'IL-5 chez le cobaye.

### - Inhibition de la sécrétion de cytokines

L'activité des produits de l'invention sur la sécrétion de cytokines par des mononucléaires humains a été mesurée *in vitro* selon une méthode décrite par Konno S. *et al.* (1994) Eur. J. Pharmacol. 264 : 265-268 et Endo H. *et al.* (1993) Int. Arch. Allergy Immunol. 101 : 425-430 pour les interleukines, et par Semmler J. *et al*. (1993) Int. J. Immunopharmac. 15 : 409-413 et Verghese M. W. *et al*. (1995) J. Pharmacol. Exp. Ther. 272 : 1313-1320 pour le TNFα. Le dosage des interleukines IL-2, IL-4, IL-5 et du TNFα est réalisé par une méthode immuno-enzymatique. On calcule la concentration CI₅₀ inhibant de 50 % la production de cytokine stimulée par la concanavaline A, la phyto-hémagglutinine ou du lipopolysaccharide.

Dans ces conditions, les produits (I) de l'invention testés ont montré une activité inhibitrice marquée, avec des CI₅₀ généralement inférieures ou égales à 10⁻⁵ mol. l⁻¹.

### - Toxicologie

La toxicité subaiguë de l'exemple 2.V a été recherchée chez le rat par voie *per os.* Administré pendant deux semaines en suspension aqueuse dans la méthylcellulose à 1%, à la dose de 100 mg/kg/j, le produit n'a montré aucune activité pouvant être reliée à un effet de toxicité.

En particulier, l'absence d'effets émétisants a été confirmée chez le chien. Les produits des exemples 4 et 9.B à la dose de 3 mg/kg par voie i.v. n'ont pas montré d'effets émétisants.

Ces résultats démontrent l'activité anti-inflammatoire et/ou immuno-suppressive des produits de l'invention. Les produits de l'invention seront donc particulièrement utiles pour le traitement ou la prévention :
- des pathologies allergiques, et notamment l'asthme, la dermatite atopique ;
- des pathologies inflammatoires notamment au niveau de la bronche, mais aussi la polyarthrite rhumatoïde, et également les affections intestinales inflammatoires (rectocolite hémorragique et maladie de Crohn) ;
y compris lorsqu'il existe une composante auto-immune.

### Partie galénique

Les produits de l'invention sont administrés sous forme de compositions appropriées à la nature et à l'importance de l'affection à traiter. La posologie journalière chez l'homme est habituellement comprise entre 2 mg et 1 g de produit qui peut être absorbé en une ou plusieurs prises. Les compositions sont préparées sous des formes compatibles avec la voie d'administration envisagée, comme par exemple les comprimés, dragées, capsules, collutoires, aérosols, poudres pour inhalation, suppositoires, gels ou suspensions. Ces compositions sont préparées par des méthodes courantes pour l'homme de l'art et comprennent de 0,5 à 60 % en poids de principe actif (composé de formule I) et 40 à 99,5 % en poids de véhicule pharmaceutique approprié et compatible avec le principe actif et la forme physique de la composition envisagée. A titre d'exemple, on présente la composition et la préparation de comprimés contenant un composé de l'invention :

| | |
|---|---|
| Substance active de formule (I) | 1 à 75 mg |
| Lactose | 124 à 74 m g |
| Cellulose microcristalline | 36 à 60 mg |
| Polyvinylpyrrolidone | 6 mg |
| Carboxyméthylamidon sodique | 8 mg |
| Stéarate de magnésium | 1 mg |

Mélanger la substance active, le lactose, la cellulose microcristalline et le carboxyméthylamidon. Mouiller et granuler à l'aide d'une solution aqueuse ou alcoolique de polyvinylpyrrolidone de concentration adaptée. Sécher et calibrer le granulé. Mélanger de façon homogène le stéarate de magnésium.Comprimer à raison de 200 mg par comprimé.

## Revendications

1. Utilisation d'un [1,4]diazépino[6,7,1-*hi*]indole pour préparer un médicament permettant de traiter des affections relevant d'une thérapie par un inhibiteur de phosphodiestérases IV.

2. Utilisation selon la revendication 1 de diazépino-indoles de formule 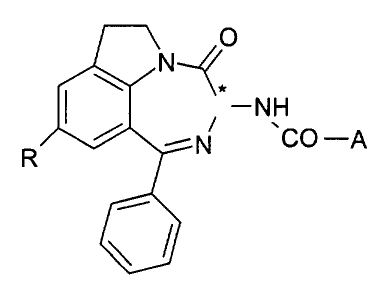 dans laquelle :
- R est hydrogène, alkyle inférieur ou alkoxy inférieur ;
- A est aryle, hétéroaryle azoté, ou hétéroaryle soufré, chacun éventuellement substitué par un à trois groupes indépendamment choisis parmi halogène, alkyle inférieur, haloalkyle, alkoxy inférieur, hydroxy, acétoxy, amino, *t*-butoxycarbonylamino, cycloalkylcarbonylamino, ou acétamido ; de leurs formes racémiques, de leurs isomères de configuration déterminée par le carbone en position 3 du noyau diazépino-indol-4-one, et de leurs sels pharmacologiquement acceptables.

3. Utilisation selon les revendications 1 ou 2 d'un diazépino-indole de formule 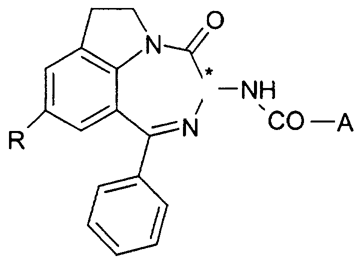 dans laquelle :
- R est hydrogène, alkyle inférieur ou alkoxy inférieur ;
- A est aryle, hétéroaryle azoté, ou hétéroaryle soufré, chacun éventuellement substitué par un à trois groupes indépendamment choisis parmi halogène, alkyle inférieur, haloalkyle, alkoxy inférieur, hydroxy, acétoxy, amino, *t*-butoxycarbonylamino, cycloalkylcarbonylamino, ou acétamido ; de leurs formes racémiques, de leurs isomères de configuration déterminée par le carbone en position 3 du noyau diazépino-indol-4-one, et de leurs sels pharmacologiquement acceptables, sous réserve que, lorsque R est hydrogène :
*i)* A ne représente pas le radical 2-indolyle, ni un radical phényle substitué par de un à trois groupes alkoxy ;
*ii)* pour les formes racémiques ou de configuration (S), A ne représente pas un radical phényle substitué par :
un halogène,
un halogène et un groupe amino,
un groupe haloalkyle, ou
de un à trois groupes alkoxy.

4. Utilisation selon l'une des revendications 1 à 3 **caractérisée en ce que** le médicament permet de prévenir ou de traiter des pathologies inflammatoires telles que l'asthme ou 1a polyarthrite rhumatoïde.

5. Diazépinoindoles de formule 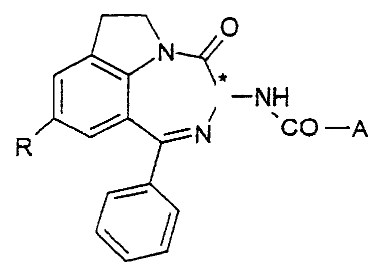 dans laquelle :
- R est hydrogène, alkyle inférieur ou alkoxy inférieur ;
- A est aryle, hétéroaryle azoté, ou hétéroaryle soufré, chacun éventuellement substitué par un à trois groupes indépendamment choisis parmi halogène, alkyle inférieur, haloalkyle, alkoxy inférieur, hydroxy, acétoxy, amino, *t*-butoxycarbonylamino, cycloalkylcarbonylamino ou acétamido ; leurs formes racémiques et leurs isomères de configuration déterminée par le carbone en position 3 du noyau diazépino-indol-4-one ainsi que leurs sels pharmacologiquement acceptables, sous réserve que, lorsque R est hydrogène :
*i)* A ne représente pas le radical 2-indolyle, ni un radical phényle substitué par de un à trois groupes alkoxy ;
*ii)* pour les formes racémiques ou de configuration (S), A ne représente pas un radical phényle substitué par :
un halogène,
un halogène et un groupe amino,
un groupe haloalkyle, ou
de un à trois groupes alkoxy.

6. Diazépino-indoles selon la revendication 5 **caractérisés en ce que** la configuration absolue du carbone en position alpha par rapport au carbonyle du cycle diazépine est (R).

7. Diazépino-indoles selon la revendication 5 ou 6 **caractérisés en ce que** A est phényle, naphtyle, pyridyle, thiényle, pyrazinyle, pyrimidyle, indolyle, quinolyle, isoquinolyle, pyrazolo-triazinyle, imidazo-pyridyle ou imidazo-pyrimidyle, chacun éventuellement substitué par un à trois groupes indépendamment choisis parmi halogène, alkyle inférieur, haloalkyle, alkoxy inférieur, hydroxy, acétoxy, amino, t-butoxycarbonylamino, cycloalkylcarbonylamino, ou acétamido.

8. Diazépino-indoles selon l'une des revendications 5 à 7 **caractérisés en ce que** R est alkyle inférieur ou alkoxy inférieur.

9. Diazépino-indoles selon l'une des revendications 5 à 8 **caractérisés en ce que** R est méthyle ou méthoxy.

10. Diazépino-indoles suivant la revendication 5 qui sont :
a) le (3R)-N-(4-oxo-1-phényl-3,4,6,7-tétrahydro-[1,4]diazépino[6,7,1-hi]indol-3-yl)-isonicotinamide,
b) le (3R)-isoquinoline-3-carboxylique acide (4-oxo-1-phényl-3,4,6,7-tétrahydro-[1,4]diazépino[6,7,1-hi]indol-3-yl)-amide,
c) le (3R)-4,7-diméthyl-pyrazolo[5,1-c][1,2,4]triazine-3-carboxylique acide (4-oxo-1-phényl-3,4,6,7-tétrahydro-[1,4]diazépino[6,7,1-hi]indol-3-yl)-amide,
d) le (3R)-isoquinoline-3-carboxylique acide (9-méthyl-4-oxo-1-phényl-3,4,6,7-tétrahydro-[1,4]diazépino[6,7,1-hi] indol-3 -yl)-amide,
e) le (3R)-4-chloro-N-(9-méthoxy-4-oxo-1-phényl-3,4,6,7-tétrahydro-[1,4]diazépino-[6,7,1-hi] indol-3-yl)-benzamide,
f) le (3R)-quinoline-3-carboxylique acide (9-méthoxy-4-oxo-1-phényl-3,4,6,7-tétrahydro-[1,4]diazépino[6,7,1-hi]indol-3-yl)-amide,
g) le (3R)-quinoline-6-carboxylique acide (9-méthoxy-4-oxo-1-phényl-3,4,6,7-tétrahydro[1,4]diazépino[6,7,1-hi]indol-3-yl)-amide,
h) le (3R)-3-chloro-4-méthoxy-N-(4-oxo-1-phényl-3,4,6,7-tétrahydro-[1,4]diazépino-[6,7,1-hi] indol-3-yl)-benzamide,
i) le (3R)-4-amino-3,5-dichloro-N-(4-oxo-1-phényl-3,4,6,7-tétrahydro-[1,4]diazépino-[6,7,1-hi]indol-3-yl)-benzamide,
j) le (3R)-5,6-dichloro-N-(4-oxo-1-phényl-3,4,6,7-tétrahydro-[1,4]diazépino[6,7,1-hi]indol-3-yl)-nicotinamide,
k) le (3R)-2-méthoxy-N-(9-méthyl-4-oxo-1-phényl-3,4,6,7-tétrahydro[1,4]diazépino-[6,7,1hi]indol-3-yl)-benzamide,
1) le (3R)-N-(9-méthyl-4-oxo-1-phényl-3,4,6,7-tétrahydro[1,4]diazépino[6,7,1-hi]indol-3-yl)-isonicotinamide,
m) le (3R)-4,7-diméthyl-pyrazolo[5,1-c] [1,2,4] triazine-3-carboxylique acide (9-méthyl-4-oxo-1-phényl-3,4,6,7-tétrahydro-[1,4]diazépino[6,7,1-hi]indol-3-yl)-amide.

11. Diazépino-indoles de formule: 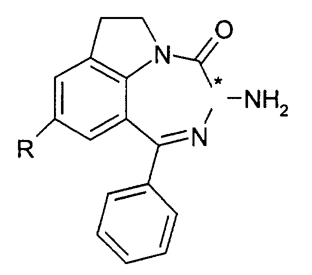 dans laquelle R est méthyle ou méthoxy.

12. Composition pharmaceutique **caractérisée en ce qu'**elle comprend à titre de principe actif au moins un diazépino-indole tel que défini à la revendication 5, en association le cas échéant avec un véhicule pharmaceutiquement acceptable.

13. Composition pharmaceutique selon la revendication 12 **caractérisée en ce que** la configuration absolue du carbone en position alpha par rapport au carbonyle du cycle diazépine du diazépino-indole est (R).

14. Composition pharmaceutique selon la revendication 12 ou 13 **caractérisée en ce que** dans le diazépino-indole le groupe R est méthyle ou méthoxy.

15. Composition pharmaceutique selon l'une des revendications 12 à 14 **caractérisée en ce que** le diazépino-indole est :
a) le (3R)-N-(4-oxo-1-phényl-3,4,6,7-tétrahydro-[1,4]diazépino[6,7,1-hi]indol-3-yl)-isonicotinamide,
b) le (3R)-isoquinoline-3-carboxylique acide (4-oxo-1-phényl-3,4,6,7-tétrahydro-[1,4]diazépino[6,7,1-hi]indol-3-yl)-amide,
c) le (3R)-4,7-diméthyl-pyrazolo[5,1-c][1,2,4]triazine-3-carboxylique acide (4-oxo-1-phényl-3,4,6,7-tétrahydro-[1,4]diazépino[6,7,1-hi]indol-3-yl)-amide,
d) le (3R)-isoquinoline-3-carboxylique acide (9-méthyl-4-oxo-1-phényl-3,4,6,7-tétrahydro-[1,4]diazépino[6,7,1-hi]indol-3-yl)-amide,
e) le (3R)-4-chloro-N-(9-méthoxy-4-oxo-1-phényl-3,4,6,7-tétrahydro-[1,4]diazépino[6,7,1-hi]indol-3-yl)-benzamide,
f) le (3R)-quinoline-3-carboxylique acide (9-méthoxy-4-oxo-1-phényl-3,4,6,7-tétrahydro-[1,4]diazépino[6,7,1-hi]indol-3-yl)-amide,
g) le (3R)-quinoline-6-carboxylique acide (9-méthoxy-4-oxo-1-phényl-3,4,6,7-tétrahydro[1,4]diazépino[6,7,1-hi]indol-3-yl)-amide,
h) le (3R)-3-chloro-4-méthoxy-N-(4-oxo-1-phényl-3,4,6,7-tétrahydro-[1,4]diazépino[6,7,1-*hi*]indol-3-yl)-benzamide,
i) le (3R)-4-amino-3,5-dichloro-N-(4-oxo-1-phényl-3,4,6,7-tétrahydro-[1,4]diazépino[6,7,1-*hi*]indol-3-yl)-benzamide,
j) le (3R)-5,6-dichloro-N-(4-oxo-1-phényl-3,4,6,7-tétrahydro-[1,4]diazépino[6,7,1-*hi*]indol-3-yl)-nicotinamide,
k) le (3R)-2-méthoxy-N-(9-méthyl-4-oxo-1-phényl-3,4,6,7-tétrahydro-[1,4]diazépino[6,7,1-*hi*]indol-3-yl)-benzamide,
1) le (3R)-N-(9-méthyl-4-oxo-1-phényl-3,4,6,7-tétrahydro-[1,4]diazépino[6,7,1-*hi*]indol-3-yl)-isonicotinamide, ou
m) le (3R)-4,7-diméthyl-pyrazolo[5,1-c][1,2,4]triazine-3-carboxylique acide (9-méthyl-4-oxo-1-phényl-3,4,6,7-tétrahydro-[1,4]diazépino[6,7,1-hi]indol-3-yl)-amide.

16. Procédé de préparation de diazépino-indoles selon la revendication 5 **caractérisé en ce qu'**il consiste à mettre à réagir une amine racémique ou optiquement active de formule : 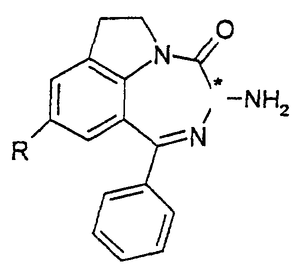 dans laquelle R est hydrogène, alkyle inférieur ou alkoxy inférieur sur un dérivé d'acide carboxylique de formule : 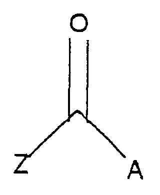 dans laquelle :
- A est aryle, hétéroaryle azoté, ou hétéroaryle soufré, chacun éventuellement substitué par un à trois groupes indépendamment choisis parmi halogène, alkyle inférieur, haloalkyle, alkoxy inférieur, hydroxy, acétoxy, amino, *t*-butoxycarbonylamino, cycloalkylcarbonylamino ou acétamido, sous réserve que, lorsque R est hydrogène :
*i)* A ne représente pas le radical 2-indolyle; ni un radical phényle substitué par de un à 3 groupes alkoxy;
*ii)* pour les formes racémiques ou de configuration (S), A ne représente pas un radical phényle substitué par :
un halogène,
un halogène et un groupe amino,
un groupe haloalkyle, ou
de un à trois groupes alkoxy.
- Z est un halogène, un groupe hydroxy, un groupe azido, un groupe imidazol-1-yle, un groupe -O-CO-Z₁, Z₁ pouvant être, outre A, un radical alkyle encombré comportant de 3 à 6 atomes de carbone, enfin Z₁ pouvant être un groupe O-Z₂, Z₂ étant un groupe aromatique comportant un ou deux cycles substitué par un ou plusieurs radicaux nitro ou halogènes, pour obtenir un diazépino-indole de formule : 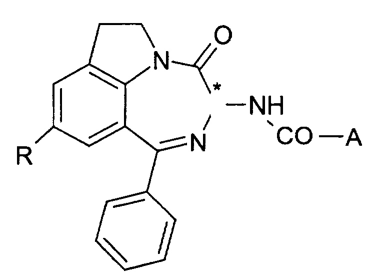
dans laquelle R et A ont la même signification qu'indiqué ci-dessus.

17. Procédé selon la revendication 16 **caractérisé en ce que** R est méthyle ou méthoxy.

18. Procédé de préparation de diazépino-indoles selon la revendication 11 **caractérisé en ce qu'**il consiste :
a) pour préparer un diazépino-indole selon la revendication 11 sous forme racémique :
1) à réduire un dérivé indole de formule : 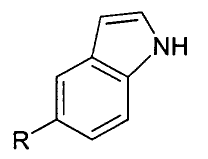 dans laquelle R est méthyle ou méthoxy en l'indoline correspondante de formule : 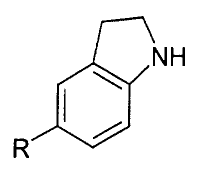
2) condenser cette indoline avec le benzonitrile en présence d'acide de Lewis pour donner, après hydrolyse, 1a benzophénone de formule : 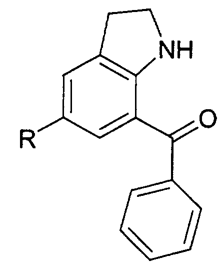
3) cycliser cette benzophénone avec le glycinate d'éthyle pour donner un diazépino-indole de formule : 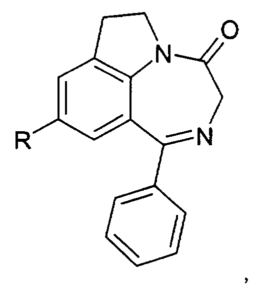 et
4) aminer en position alpha du carbonyle le diazépino-indole obtenu au stade 3) précédent pour obtenir un diazépino-indole de formule (II) 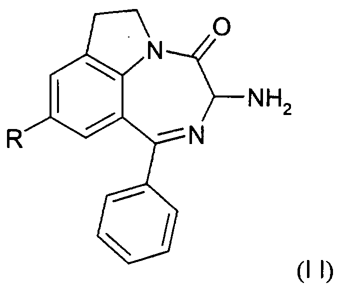 dans lequel R est méthyle ou méthoxy,
b) pour préparer un diazépino-indole selon la revendication 11 optiquement actif :
- à condenser un composer (II) racémique avec un dérivé d'acide alpha aminé appartenant à la série D ou à la série L et dans lequel la fonction amine est protégée par un groupement facilement labile, de préférence le groupement tertiobutyloxycarbonyle ; le composé obtenu est déprotégé par hydrolyse, de préférence en milieu acide en présence d'acide trifluoroacétique, et le produit obtenu est séparé en ses diastéréoisomères par chromatographie ; on obtient les deux isomères de l'amine condensée avec l'acide aminé ; par dégradation d'Edman, on revient ensuite aux deux énantiomères de l'amine (II) ; ou encore,
- à dissoudre un composé (II) racémique dans une solution d'acide optiquement actif, comme par exemple un énantiomère des acides mandélique, dibenzoyltartrique, di-p-toluyltartrique, camphosulfonique, p-nitrobenzoylglutamique, ou tartrique, pour former deux sels diastéréoisomères, puis par différence de solubilité, en cristalliser sélectivement un dans un solvant approprié.

## Patentansprüche

1. Verwendung eines [1,4]Diazepino[6,7,1-*hi*]indols zur Herstellung eines Medikaments, das die Behandlung von Erkrankungen ermöglicht, die in das Gebiet einer Therapie durch einen Inhibitor von Phosphodiesterasen IV fallen.

2. Verwendung gemäß Anspruch 1 von Diazepino-indolen der Formel 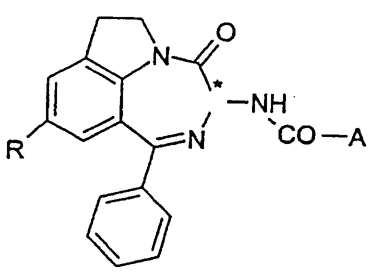 worin:
- R Wasserstoff, niederes Alkyl oder niederes Alkoxy ist;
- A Aryl, stickstoffhaltiges Heteroaryl oder schwefelhaltiges Heteroaryl ist, wobei jedes gegebenenfalls mit einer bis drei Gruppen substituiert ist, die unabhängig ausgewählt sind unter Halogen, niederem Alkyl, Halogenalkyl, niederem Alkoxy, Hydroxy, Acetoxy, Amino, t-Butoxycarbonylamino, Cycloalkylcarbonylamino oder Acetamido;
ihrer racemischen Formen, ihrer Isomere mit einer Konfiguration, die festgelegt ist durch den Kohlenstoff in Position 3 des Diazepino-indol-4-on-Rings, und ihrer pharmakologisch annehmbaren Salze.

3. Verwendung gemäß den Ansprüchen 1 oder 2 eines Diazepino-indols der Formel 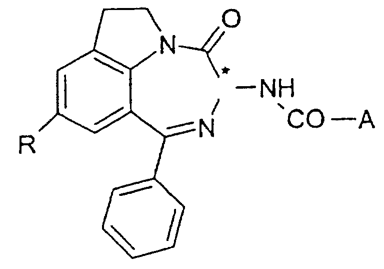 worin:
- R Wasserstoff, niederes Alkyl oder niederes Alkoxy ist;
- A Aryl, stickstoffhaltiges Heteroaryl oder schwefelhaltiges Heteroaryl ist, wobei jedes gegebenenfalls mit einer bis drei Gruppen substituiert ist, die unabhängig ausgewählt sind unter Halogen, niederem Alkyl, Halogenalkyl, niederem Alkoxy, Hydroxy, Acetoxy, Amino, t-Butoxycarbonylamino, Cycloalkylcarbonylamino oder Acetamido;
seiner racemischen Formen, seiner Isomere mit einer Konfiguration, die festgelegt ist durch den Kohlenstoff in Position 3 des Diazepino-indol-4-on-Rings, und seiner pharmakologisch annehmbaren Salze unter dem Vorbehalt, dass, wenn R Wasserstoff ist:
*i)* A weder den 2-Indolylrest noch einen mit einer bis drei Alkoxygruppen substituierten Phenylrest darstellt;
*ii)* für die racemischen Formen oder die Formen mit (S)-Konfiguration A keinen Phenylrest darstellt, der substituiert ist mit:
einem Halogen,
einem Halogen und einer Aminogruppe,
einer Halogenalkylgruppe oder
einer bis drei Alkoxygruppen.

4. Verwendung gemäß einem der Ansprüche 1 bis 3, **dadurch gekennzeichnet, dass** das Medikament die Vorbeugung oder Behandlung von entzündlichen Krankheitsbildern, wie Asthma oder rheumatoide Polyarthritis, ermöglicht.

5. Diazepinoindole der Formel 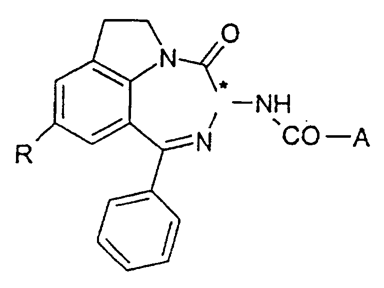 worin:
- R Wasserstoff, niederes Alkyl oder niederes Alkoxy ist;
- A Aryl, stickstoffhaltiges Heteroaryl oder schwefelhaltiges Heteroaryl ist, wobei jedes gegebenenfalls mit einer bis drei Gruppen substituiert ist, die unabhängig ausgewählt sind unter Halogen, niederem Alkyl, Halogenalkyl, niederem Alkoxy, Hydroxy, Acetoxy, Amino, t-Butoxycarbonylamino, Cycloalkylcarbonylamino oder Acetamido;
ihre racemischen Formen und ihre Isomere mit einer Konfiguration, die festgelegt ist durch den Kohlenstoff in Position 3 des Diazepino-indol-4-on-Rings, sowie ihre pharmakologisch annehmbaren Salze unter dem Vorbehalt, dass, wenn R Wasserstoff ist:
*i)* A weder den 2-Indolylrest noch einen mit einer bis drei Alkoxygruppen substituierten Phenylrest darstellt;
*ii)* für die racemischen Formen oder die Formen mit (S)-Konfiguration A keinen Phenylrest darstellt, der substituiert ist mit:
einem Halogen,
einem Halogen und einer Aminogruppe,
einer Halogenalkylgruppe oder
einer bis drei Alkoxygruppen.

6. Diazepino-indole gemäß Anspruch 5, **dadurch gekennzeichnet, dass** die absolute Konfiguration des Kohlenstoffs in alpha-Position zur Carbonylgruppe des Diazepinrings (R) ist.

7. Diazepino-indole gemäß Anspruch 5 oder 6, **dadurch gekennzeichnet, dass** A Phenyl, Naphthyl, Pyridyl, Thienyl, Pyrazinyl, Pyrimidyl, Indolyl, Chinolyl, Isochinolyl, Pyrazolo-triazinyl, Imidazo-pyridyl oder Imidazo-pyrimidyl ist, wobei jedes gegebenenfalls mit einer bis drei Gruppen substituiert ist, die unabhängig ausgewählt sind unter Halogen, niederem Alkyl, Halogenalkyl, niederem Alkoxy, Hydroxy, Acetoxy, Amino, t-Butoxycarbonylamino, Cycloalkylcarbonylamino oder Acetamido.

8. Diazepino-indole gemäß einem der Ansprüche 5 bis 7, **dadurch gekennzeichnet, dass** R niederes Alkyl oder niederes Alkoxy ist.

9. Diazepino-indole gemäß einem der Ansprüche 5 bis 8, **dadurch gekennzeichnet, dass** R Methyl oder Methoxy ist.

10. Diese Diazepino-indole gemäß Anspruch 5:
a) (3R)-N-(4-Oxo-1-phenyl-3,4,6,7-tetrahydro-[1,4]diazepino[6,7,1-hi]indol-3-yl)-isonicotinamid,
b) (3R)-lsochinolin-3-carbonsäure-(4-oxo-1-phenyl-3,4,6,7-tetrahydro-[1,4]diazepino[6,7,1-hi]indol-3-yl)-amid,
c) (3R)-4,7-Dimethyl-pyrazolo[5,1-c][1,2,4]triazin-3-carbonsäure-(4-oxo-1-phenyl-3,4,6,7-tetrahydro-[1,4]diazepino[6,7,1-hi]indol-3-yl)-amid,
d) (3R)-Isochinolin-3-carbonsäure-(9-methyl-4-oxo-1 -phenyl-3,4,6,7-tetrahydro-[1,4]diazepino[6,7,1-hi]indol-3-yl)-amid,
e) (3R)-4-Chlor-N-(9-methoxy-4-oxo-1-phenyl-3,4,6,7-tetrahydro-[1,4]diazepino-[6,7,1-hi]indol-3-yl)-benzamid,
f) (3R)-Chinolin-3-carbonsäure-(9-methoxy-4-oxo-1-phenyl-3,4,6,7-tetrahydro-[1,4]diazepino[6,7,1-hi]indol-3-yl)-amid,
g) (3R)-Chinolin-6-carbonsäure-(9-methoxy-4-oxo-1-phenyl-3,4,6,7-tetrahydro-[1,4]diazepino[6,7,1-hi]indol-3-yl)-amid,
h) (3R)-3-Chlor-4-methoxy-N-(4-oxo-1-phenyl-3,4,6,7-tetrahydro-[1,4]diazepino-[6,7,1-hi]indol-3-yl)-benzamid,
i) (3R)-4-Amino-3,5-dichlor-N-(4-oxo-1-phenyl-3,4,6,7-tetrahydro-[1,4]diazepino-[6,7,1-hi]indol-3-yl)-benzamid,
j) (3R)-5,6-Dichlor-N-(4-oxo-1-phenyl-3,4,6,7-tetrahydro-[1,4]diazepino[6,7,1-hi]-indol-3-yl)-nicotinamid,
k) (3R)-2-Methoxy-N-(9-methyl-4-oxo-1-phenyl-3,4,6,7-tetrahydro-[1,4]diazepino-[6,7,1-hi]indol-3-yl)-benzamid,
l) (3R)-N-(9-Methyl-4-oxo-1-phenyl-3,4,6,7-tetrahydro-[1,4]diazepino[6,7,1-hi]indol-3-yl)-isonicotinamid,
m) (3R)-4,7-Dimethyl-pyrazolo[5,1-c][1,2,4]triazin-3-carbonsäure-(9-methyl-4-oxo-1-phenyl-3,4,6,7-tetrahydro-[1,4]diazepino[6,7,1-hi]indol-3-yl)-amid.

11. Diazepino-indole der Formel: 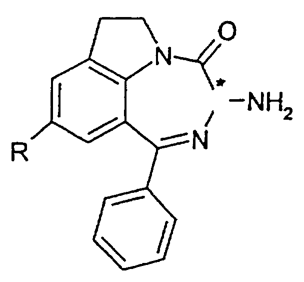 worin R Methyl oder Methoxy ist.

12. Pharmazeutische Zusammensetzung, **dadurch gekennzeichnet, dass** sie als Wirkstoff wenigstens ein Diazepino-indol, wie in Anspruch 5 definiert, gegebenenfalls in Kombination mit einem pharmazeutisch annehmbaren Träger umfasst.

13. Pharmazeutische Zusammensetzung gemäß Anspruch 12, **dadurch gekennzeichnet, dass** die absolute Konfiguration des Kohlenstoffs in alpha-Position zur Carbonylgruppe des Diazepinrings des Diazepino-indols (R) ist.

14. Pharmazeutische Zusammensetzung gemäß Anspruch 12 oder 13, **dadurch gekennzeichnet, dass** die Gruppe R in dem Diazepino-indol Methyl oder Methoxy ist.

15. Pharmazeutische Zusammensetzung gemäß einem der Ansprüche 12 bis 14, **dadurch gekennzeichnet, dass** das Diazepinoindol ist:
a) (3R)-N-(4-Oxo-1-phenyl-3,4,6,7-tetrahydro-[1,4]diazepino[6,7,1-hi]indol-3-yl)-isonicotinamid,
b) (3R)-Isochinolin-3-carbonsäure-(4-oxo-1-phenyl-3,4,6,7-tetrahydro-[1,4]diazepino[6,7,1-hi]indol-3-yl)-amid,
c) (3R)-4,7-Dimethyl-pyrazolo[5,1-c][1,2,4]triazin-3-carbonsäure-(4-oxo-1-phenyl-3,4,6,7-tetrahydro-[1,4]diazepino[6,7,1-hi]indol-3-yl)-amid,
d) (3R)-lsochinolin-3-carbonsäure-(9-methyl-4-oxo-1-phenyl-3,4,6,7-tetrahydro-[1,4]-diazepino[6,7,1-hi]indol-3-yl)-amid,
e) (3R)-4-Chlor-N-(9-methoxy-4-oxo-1-phenyl-3,4,6,7-tetrahydro-[1,4]diazepino-[6,7,1-hi]indol-3-yl)-benzamid,
f) (3R)-Chinolin-3-carbonsäure-(9-methoxy-4-oxo-1-phenyl-3,4,6,7-tetrahydro-[1,4]diazepino[6,7,1-hi]indol-3-yl)-amid,
g) (3R)-Chinolin-6-carbonsäure-(9-methoxy-4-oxo-1-phenyl-3,4,6,7-tetrahydro-[1,4]diazepino[6,7,1-hi]indol-3-yl)-amid,
h) (3R)-3-Chlor-4-methoxy-N-(4-oxo-1-phenyl-3,4,6,7-tetrahydro-[1,4]diazepino-[6,7,1-*hi*]indol-3-yl)-benzamid,
i) (3R)-4-Amino-3,5-dichlor-N-(4-oxo-1-phenyl-3,4,6,7-tetrahydro-[1,4]diazepino-[6,7,1-*hi*]indol-3-yl)-benzamid,
j) (3R)-5,6-Dichlor-N-(4-oxo-1 -phenyl-3,4,6,7-tetrahydro-[1,4]diazepino[6,7,1-*hi*]-indol-3-yl)-nicotinamid,
k) (3R)-2-Methoxy-N-(9-methyl-4-oxo-1-phenyl-3,4,6,7-tetrahydro-[1,4]diazepino-[6,7,1-*hi*]indol-3-yl)-benzamid,
l) (3R)-N-(9-Methyl-4-oxo-1-phenyl-3,4,6,7-tetrahydro-[1,4]diazepino[6,7,1-*hi*]indol-3-yl)-isonicotinamid oder
m) (3R)-4,7-Dimethyl-pyrazolo[5,1-c][1,2,4]triazin-3-carbonsäure-(9-methyl-4-oxo-1-phenyl-3,4,6,7-tetrahydro-[1,4]diazepino[6,7,1-hi]indol-3-yl)-amid.

16. Verfahren zur Herstellung von Diazepino-indolen gemäß Anspruch 5, **dadurch gekennzeichnet, dass** es darin besteht, ein racemisches oder optisch aktives Amin der Formel: 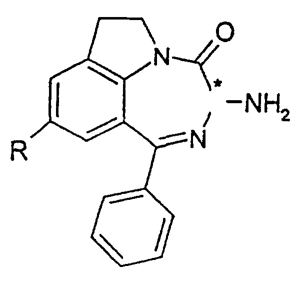 worin R Wasserstoff, niederes Alkyl oder niederes Alkoxy ist, mit einem Carbonsäurederivat der Formel: 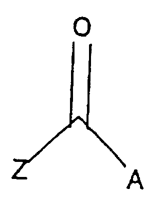 zur Reaktion zu bringen, worin:
- A Aryl, stickstoffhaltiges Heteroaryl oder schwefelhaltiges Heteroaryl ist, wobei jedes gegebenenfalls mit einer bis drei Gruppen substituiert ist, die unabhängig ausgewählt sind unter Halogen, niederem Alkyl, Halogenalkyl, niederem Alkoxy, Hydroxy, Acetoxy, Amino, t-Butoxycarbonylamino, Cycloalkylcarbonylamino oder Acetamido unter dem Vorbehalt, dass, wenn R Wasserstoff ist:
*i)* A weder den 2-Indolylrest noch einen mit einer bis 3 Alkoxygruppen substituierten Phenylrest darstellt;
*ii)* für die racemischen Formen oder die Formen mit (S)-Konfiguration A keinen Phenylrest darstellt, der substituiert ist mit:
einem Halogen,
einem Halogen und einer Aminogruppe,
einer Halogenalkylgruppe oder
einer bis drei Alkoxygruppen;
- Z ein Halogen, eine Hydroxygruppe, eine Azidogruppe, eine Imidazol-1-ylgruppe, eine Gruppe -O-CO-Z₁ ist, wobei Z₁ außer A ein sperriger Alkylrest mit 3 bis 6 Kohlenstoffatomen sein kann, Z₁ schließlich eine Gruppe O-Z₂ sein kann, wobei Z₂ eine aromatische Gruppe mit einem oder zwei Ringen ist, die mit einem oder mehreren Nitroresten oder Halogenen substituiert ist, um ein Diazepino-indol der Formel: 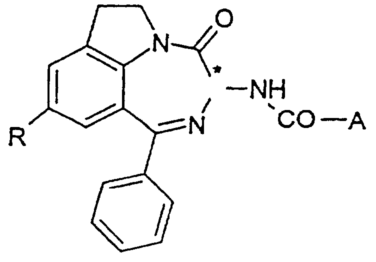 zu erhalten, worin R und A die gleiche Bedeutung wie oben angegeben haben.

17. Verfahren gemäß Anspruch 16, **dadurch gekennzeichnet, dass** R Methyl oder Methoxy ist.

18. Verfahren zur Herstellung von Diazepino-indolen gemäß Anspruch 11, **dadurch gekennzeichnet, dass** es darin besteht:
a) um ein Diazepino-indol gemäß Anspruch 11 in racemischer Form herzustellen:
1) ein Indolderivat der Formel: 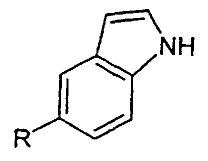 worin R Methyl oder Methoxy ist, zu dem entsprechenden Indolin der Formel: 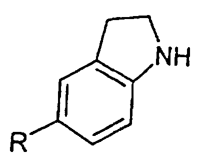 zu reduzieren,
2) dieses Indolin mit Benzonitril in Gegenwart einer Lewis-Säure zu kondensieren, um nach Hydrolyse das Benzophenon der Formel: 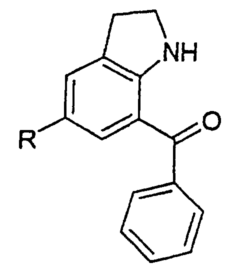 zu ergeben,
3) dieses Benzophenon mit Ethylglycinat zu cyclisieren, um ein Diazepinoindol der Formel: 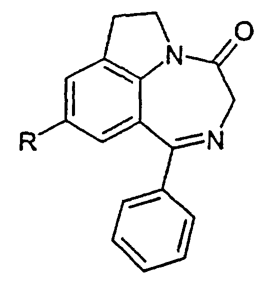 zu ergeben, und
4) das in der vorherigen Stufe 3) erhaltene Diazepino-indol in alpha-Position zur Carbonylgruppe zu aminieren, um ein Diazepino-indol der Formel (II) 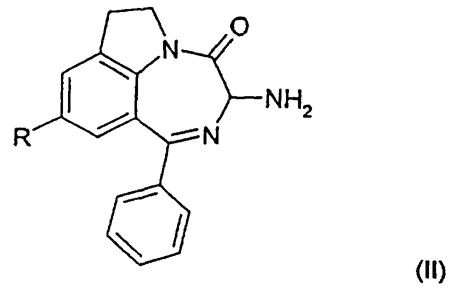 zu erhalten, worin R Methyl oder Methoxy ist,
b) um ein optisch aktives Diazepino-indol gemäß Anspruch 11 herzustellen:
- eine racemische Verbindung (II) mit einem alpha-Aminosäurederivat aus der D-Reihe oder der L-Reihe, worin die Aminfunktion durch eine leicht labile Gruppe, vorzugsweise die tert.-Butyloxycarbonylgruppe, geschützt ist, zu kondensieren; die Schutzgruppen werden von der erhaltenen Verbindung durch Hydrolyse, vorzugsweise in saurem Medium in Gegenwart von Trifluoressigsäure, entfernt und das erhaltene Produkt wird durch Chromatographie in seine Diastereoisomere aufgetrennt; man erhält die beiden Isomere des mit der Aminosäure kondensierten Amins; durch Edman-Abbau kommt man dann auf die beiden Enantiomere des Amins (II) zurück; oder auch
- eine racemische Verbindung (II) in einer Lösung einer optisch aktiven Säure, wie beispielsweise ein Enantiomer der Mandelsäure, Dibenzoylweinsäure, Di-p-toluylweinsäure, Camphersulfonsäure, p-Nitrobenzoylglutaminsäure oder Weinsäure, zu lösen, um zwei diastereoisomere Salze zu bilden, dann durch Löslichkeitsunterschied eines davon in einem geeigneten Lösungsmittel selektiv auszukristallisieren.

## Claims

1. Use of a [1,4]diazepino[6,7,1-hi]indole to prepare a medicament enabling the treatment of disorders for which therapy by a phosphodiesterase IV inhibitor is relevant.

2. Use according to Claim 1 of diazepinoindoles of formula 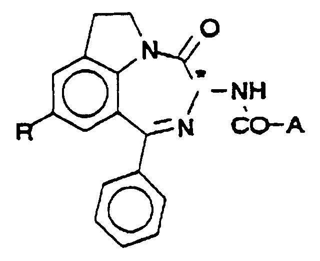 in which:
- R is hydrogen, lower alkyl or lower alkoxy;
- A is aryl, nitrogen-containing heteroaryl or sulphur-containing heteroaryl, each of which is optionally substituted by from one to three groups which are chosen independently from halogen, lower alkyl, haloalkyl, lower alkoxy, hydroxyl, acetoxy, amino, t-butoxycarbonylamino, cycloalkylcarbonylamino and acetamido; of their racemic forms, of their isomers whose configuration is determined by the carbon in position 3 of the diazepinoindole-4-one ring system, and of their pharmacologically acceptable salts.

3. Use according to Claims 1 or 2 of a diazepinoindole of formula 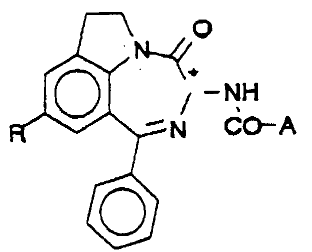 in which:
- R is hydrogen, lower alkyl or lower alkoxy;
- A is aryl, nitrogen-containing heteroaryl or sulphur-containing heteroaryl, each of which is optionally substituted by from one to three groups which are chosen independently from halogen, lower alkyl, haloalkyl, lower alkoxy, hydroxyl, acetoxy, amino, t-butoxycarbonylamino, cycloalkylcarbonylamino and acetamido; of their racemic forms, of their isomers whose configuration is determined by the carbon in position 3 of the diazepinoindole-4-one ring system, and of their pharmacologically acceptable salts, with the proviso that, when R is hydrogen:
i) A does not represent the 2-indolyl radical, or a phenyl radical substituted by from one to three alkoxy groups;
ii) for the racemic forms or those having the (S) configuration, A does not represent a phenyl radical substituted by:
a halogen,
a halogen and an amino group,
a haloalkyl group, or
from one to three alkoxy groups.

4. Use according to one of Claims 1 to 3, **characterized in that** the medicament enables the prevention or treatment of inflammatory pathologies such as asthma or rheumatoid arthritis.

5. Diazepinoindoles of formula 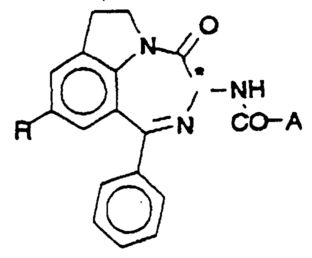 in which:
- R is hydrogen, lower alkyl or lower alkoxy;
- A is aryl, nitrogen-containing heteroaryl or sulphur-containing heteroaryl, each of which is optionally substituted by from one to three groups which are chosen independently from halogen, lower alkyl, haloalkyl, lower alkoxy, hydroxyl, acetoxy, amino, t-butoxycarbonylamino, cycloalkylcarbonylamino and acetamido; of their racemic forms, of their isomers whose configuration is determined by the carbon in position 3 of the diazepinoindole-4-one ring system, and of their pharmacologically acceptable salts, with the proviso that, when R is hydrogen:
i) A does not represent the 2-indolyl radical, or a phenyl radical substituted by from one to three alkoxy groups;
ii) for the racemic forms or those having the (S) configuration, A does not represent a phenyl radical substituted by:
a halogen,
a halogen and an amino group,
a haloalkyl group, or
from one to three alkoxy groups.

6. Diazepinoindoles according to Claim 5, **characterized in that** the absolute configuration of the carbon in the alpha position relative to the carbonyl function of the diazepine ring is (R).

7. Diazepinoindoles according to Claim 5 or 6, **characterized in that** A is phenyl, naphthyl, pyridyl, thienyl, pyrazinyl, pyrimidyl, indolyl, quinolyl, isoquinolyl, pyrazolo-triazinyl, imidazo-pyridyl or imidazo-pyrimidyl, each of which is optionally substituted by from one to three groups which are chosen independently from halogen, lower alkyl, haloalkyl, lower alkoxy, hydroxyl, acetoxy, amino, t-butoxycarbonylamino, cycloalkylcarbonylamino and acetamido.

8. Diazepinoindoles according to one of Claims 5 to 7, **characterized in that** R is lower alkyl or lower alkoxy.

9. Diazepinoindoles according to one of Claims 5 to 8, **characterized in that** R is methyl or methoxy.

10. Diazepinoindoles according to Claim 5, which are:
a) (3R)-N-(4-oxo-1-phenyl-3,4,6,7-tetrahydro-[1,4]diazepino[6,7,1-hi]indol-3-yl)isonicotinamide,
b) (3R)-N-(4-oxo-1-phenyl-3,4,6,7-tetrahydro[1,4]diazepino[6,7,1-hi]indol-3-yl)isoquinoline-3-carboxamide,
c) (3R)-N-(4-oxo-1-phenyl-3,4,6,7-tetrahydro[1,4]diazepino[6,7,1-hi]indol-3-yl)-4,7-dimethylpyrazolo[5,1c] [1,2,4]triazine-3-carboxamide,
d) (3R)-N-(9-methyl-4-oxo-1-phenyl-3,4,6,7-tetrahydro[1,4]diazepino[6,7,1-hi]indol-3-yl)isoquinoline-3-carboxamide,
e) (3R)-4-chloro-N-(9-methoxy-4-oxo-1-phenyl-3,4,6,7-tetrahydro[1,4]diazepino[6,7,1-hi]indol-3-yl)benzamide,
f) (3R)-N-(9-methoxy-4-oxo-1-phenyl-3,4,6,7-tetrahydro[1,4]diazepino[6,7,1-hi]indol-3-yl)quinoline-3-carboxamide,
g) (3R)-(9-methoxy-4-oxo-1-phenyl-3,4,6,7-tetrahydro-[1,4]diazepino[6,7,1-hi]indol-3-yl)quinoline-6-carboxamide,
h) (3R)-3-chloro-4-methoxy-N-(4-oxo-1-phenyl-3,4,6,7-tetrahydro[1,4]diazepino[6,7,1-hi]indol-3-yl)benzamide,
i) (3R)-4-amino-3,5-dichloro-N-(4-oxo-1-phenyl-3,4,6,7-tetrahydro[1,4]diazepino[6,7,1-hi]indol-3-yl)benzamide,
j) (3R)-5,6-dichloro-N-(4-oxo-1-phenyl-3,4,6,7-tetrahydro[1,4]diazepino[6,7,1-hi]indol-3-yl)nicotinamide,
k) (3R)-2-methoxy-N-(9-methyl-4-oxo-1-phenyl-3,4,6,7-tetrahydro[1,4]diazepino[6,7,1-hi]indol-3-yl)benzamide,
l) (3R)-N-(9-methyl-4-oxo-1-phenyl-3,4,6,7-tetrahydro[1,4]diazepino[6,7,1-hi]indol-3-yl)isonicotinamide,
m) (3R)-N-(9-methyl-4-oxo-1-phenyl-3,4,6,7-tetrahydro[1,4]diazepino[6,7,1-hi]indol-3-yl)-4,7-dimethylpyrazolo[5,1-c][1,2,4]triazine-3-carboxamide.

11. Diazepinoindoles of formula: 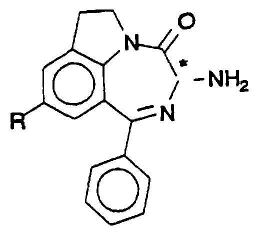 in which R is methyl or methoxy.

12. Pharmaceutical composition, **characterized in that** it comprises as active principle at least one diazepinoindole as defined in Claim 5 in combination, if appropriate, with a pharmaceutically acceptable vehicle.

13. Pharmaceutical composition according to Claim 12, **characterized in that** the absolute configuration of the carbon in the alpha position relative to the carbonyl function of the diazepine ring of the diazepinoindole is (R).

14. Pharmaceutical composition according to Claim 12 or 13, **characterized in that** in the diazepinoindole the group R is methyl or methoxy.

15. Pharmaceutical composition according to one of Claims 12 to 14, **characterized in that** the diazepinoindole is:
a) (3R)-N-(4-oxo-1-phenyl-3,4,6,7-tetrahydro-[1,4]diazepino[6,7,1-hi]indol-3-yl)-isonicotinamide,
b) (3R)-N-(4-oxo-1-phenyl-3,4,6,7-tetrahydro[1,4]diazepino[6,7,1-hi]indol-3-yl)isoquinoline-3-carboxamide,
c) (3R)-N-(4-oxo-1-phenyl-3,4,6,7-tetrahydro[1,4]diazepino[6,7,1-hi]indol-3-yl)-4,7-dimethylpyrazolo[5,1c]-[1,2,4]triazine-3-carboxamide,
d) (3R)-N-(9-methyl-4-oxo-1-phenyl-3,4,6,7-tetrahydro[1,4]diazepino[6,7,1-hi]indol-3-yl)isoquinoline-3-carboxamide,
e) (3R)-4-chloro-N-(9-methoxy-4-oxo-1-phenyl-3,4,6,7-tetrahydro[1,4]diazepino[6,7,1-hi]indol-3-yl)benzamide,
f) (3R)-N-(9-methoxy-4-oxo-1-phenyl-3,4,6,7-tetrahydro[1,4]diazepino[6,7,1-hi]indol-3-yl)quinoline-3-carboxamide,
g) (3R)-(9-methoxy-4-oxo-1-phenyl-3,4,6,7-tetrahydro-[1,4]diazepino[6,7,1-hi]indol-3-yl)quinoline-6-carboxamide,
h) (3R)-3-chloro-4-methoxy-N-(4-oxo-1-phenyl-3,4,6,7-tetrahydro[1,4]diazepino[6,7,1-hi]indol-3-yl)benzamide,
i) (3R)-4-amino-3,5-dichloro-N-(4-oxo-1-phenyl-3,4,6,7-tetrahydro[1,4]diazepino[6,7,1-hi]indol-3-yl)benzamide,
j) (3R)-5,6-dichloro-N-(4-oxo-1-phenyl-3,4,6,7-tetrahydro[1,4]diazepino[6,7,1-hi]indol-3-yl)nicotinamide,
k) (3R)-2-methoxy-N-(9-methyl-4-oxo-1-phenyl-3,4,6,7-tetrahydro[1,4]diazepino[6,7,1-hi]indol-3-yl)benzamide,
1) (3R)-N-(9-methyl-4-oxo-1-phenyl-3,4,6,7-tetrahydro[1,4]diazepino[6,7,1-hi]indol-3-yl)isonicotinamide, or
m) (3R)-N-(9-methyl-4-oxo-1-phenyl-3,4,6,7-tetrahydro[1,4]diazepino[6,7,1-hi]indol-3-yl)-4,7-dimethylpyrazolo[5,1-c][1,2,4]triazine-3-carboxamide.

16. Process for the preparation of diazepinoindoles according to Claim 5, **characterized in that** it consists in reacting a racemic or optically active amine of formula: 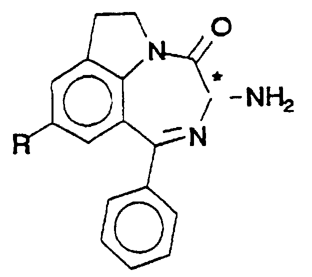 in which R is hydrogen, lower alkyl or lower alkoxy with a carboxylic acid derivative of formula: 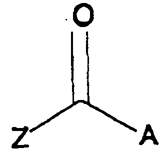 in which:
- A is aryl, nitrogen-containing heteroaryl or sulphur-containing heteroaryl, each of which is optionally substituted by from one to three groups which are chosen independently from halogen, lower alkyl, haloalkyl, lower alkoxy, hydroxyl, acetoxy, amino, t-butoxycarbonylamino, cycloalkylcarbonylamino and acetamido, with the proviso that, when R is hydrogen:
i) A does not represent the 2-indolyl radical, or a phenyl radical substituted by from one to three alkoxy groups;
ii) for the racemic forms or those having the (S) configuration, A does not represent a phenyl radical substituted by:
a halogen,
a halogen and an amino group,
a haloalkyl group, or
from one to three alkoxy groups;
- Z is a halogen, a hydroxyl group, an azido group, an imidazol-1-yl group, a group -O-CO-Z₁, in which Z₁ can be, besides A, a hindered alkyl radical containing from 3 to 6 carbon atoms, or Z₁ can be a group O-Z₂, Z₂ being an aromatic group comprising one or two rings which is substituted by one or more nitro radicals or halogens, to obtain a diazepinoindole of formula: 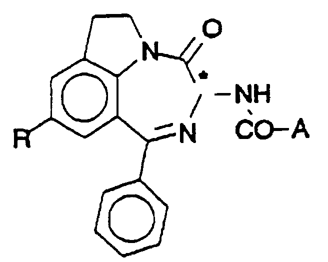 in which R and A are as defined above.

17. Process according to Claim 16, **characterized in that** R is methyl or methoxy.

18. Process for the preparation of diazepinoindoles according to Claim 11, **characterized in that** it consists:
a) in order to prepare a diazepinoindole according to Claim 11 in racemic form:
1) in reducing an indole derivative of formula: 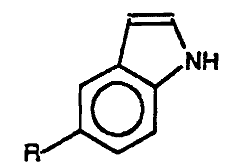 in which R is methyl or methoxy to the corresponding indoline of formula: 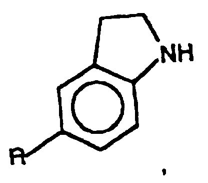
2) condensing this indoline with benzonitrile in the presence of Lewis acid in order to give, after hydrolysis, the benzophenone of formula: 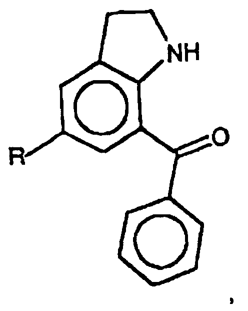
3) cyclizing this benzophenone with ethyl glycinate in order to give a diazepinoindole of formula: 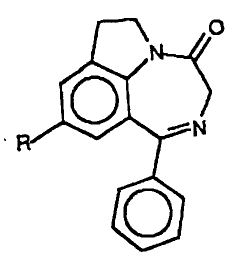 and
4) aminating the alpha position of the carbonyl function of the diazepinoindole obtained in stage 3) above in order to obtain a diazepinoindole of formula (II) 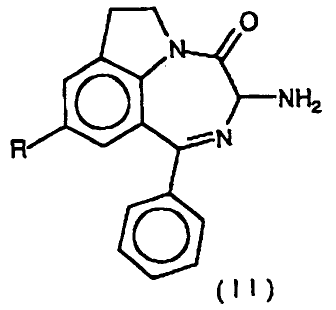 in which R is methyl or methoxy;
b) in order to prepare an optically active diazepinoindole according to Claim 11:
- in condensing a racemic compound (II) with an alpha-amino acid derivative belonging to the D series or the L series and in which the amine function is protected by a highly labile group, preferably the tert-butyloxycarbonyl group; the compound obtained is deprotected by hydrolysis, preferably in acid medium in the presence of trifluoroacetic acid, and the product obtained is separated into its diastereoisomers by chromatography; the two isomers are obtained of the amine condensed with the amino acid; Edman degradation then yields the two enantiomers of the amine (II); or alternatively
- in dissolving a racemic compound (II) in a solution of an optically active acid, for example an enantiomer of mandelic, dibenzoyltartaric, di-p-tolyltartaric, camphorsulphonic, p-nitrobenzoylglutamic or tartaric acid, to form two diastereoisomeric salts, followed, utilizing the solubility difference, by a selective crystallization of one of them in an appropriate solvent.
